# EUROPEAN PATENT SPECIFICATION

(11) **EP 3 999 637 B1**
(45) Date of publication and mention of the grant of the patent: **02.04.2025**
(21) Application number: 20840610.8
(22) Date of filing: 16.07.2020
(51) Int. Cl.: C12N 9/16, C12Q 1/42, G01N 33/68, A23L 29/00, A61K 35/741, A61P 3/04, A61P 3/10

(54) **INTESTINAL ALKALINE PHOSPHATASE-BASED TREATMENTS OF METABOLIC DISORDERS**
INTESTINALE ALKALISCHE PHOSPHATASE-BASIERTE BEHANDLUNGEN VON STOFFWECHSELSTÖRUNGEN
TRAITEMENTS DE TROUBLES MÉTABOLIQUES À BASE DE PHOSPHATASE ALCALINE INTESTINALE

(30) Priority: 18.07.2019 US 201962875536 P
(43) Date of publication of application: 25.05.2022
(73) Proprietor: Theriva Biologics, Inc., Rockville, MD 20850 (US)
(72) Inventor: FURLAN FREGUIA, Christian, Rockville, MD 20850 (US)
(74) Representative: Boxall, Sarah Jane
(86) International application number: PCT/US2020/042294
(87) International publication number: WO 2021/011754

(56) References cited:
- WO-A1-2016/093599
- WO-A1-2018/027179
- WO-A1-2018/175413
- KR-A- 20130 021 920
- US-A1- 2003 096 341
- US-A1- 2010 158 952
- US-A1- 2011 206 654
- US-A1- 2016 201 110
- US-A1- 2017 333 492
- YANG J-Y ET AL: "Gut commensal Bacteroides acidifaciens prevents obesity and improves insulin sensitivity in mice", MUCOSAL IMMUNOLOGY, vol. 10, no. 1, 27 April 2016 (2016-04-27), New York, pages 104 - 116, XP055856912, ISSN: 1933-0219, Retrieved from the Internet <URL:http://www.nature.com/articles/mi201642> DOI: 10.1038/mi.2016.42
- MALO MADHU S., MOAVEN OMEED, MUHAMMAD NUR, BISWAS BRISHTI, ALAM SAYEDA N., ECONOMOPOULOS KONSTANTINOS P., GUL SARAH SHIREEN, HAMAR: "Intestinal alkaline phosphatase promotes gut bacterial growth by reducing the concentration of luminal nucleotide triphosphates", AM J PHYSIOL GASTROINTEST LIVER PHYSIOL, vol. 306, no. 10, 10 April 2014 (2014-04-10), pages 826 - 838, XP055786243, DOI: 10.1152/ajpgi.00357.2013

## Description

### TECHNICAL FIELD

The present invention relates, *inter alia,* to combination therapies of specific gastrointestinal bacteria with therapeutic intestinal alkaline phosphatases for the treatment of metabolic disorders. The present invention further relates to compositions comprising the combination of specific gastrointestinal bacteria with therapeutic alkaline phosphatases and use of the compositions in the treatment of disease, such as metabolic disorders.

### BACKGROUND

The gut microbiome is a key component of an individual's health. For example, the gut microbiota has been shown to influence alterations in energy balance and immunity, leading to metabolic dysfunctions. Metabolic syndrome is a complex multifaceted disease, which includes obesity and diabetes. In both humans and animal models, high fat diets have been shown to cause metabolic syndrome by contributing to dysbiosis and an overall pro-inflammatory environment. Indeed, a high fat diet is associated with changes to the gut microbiota composition. Alkaline phosphatase ("APs," EC 3.1.3.1) is a hydrolase enzyme that can remove phosphate groups from various targets, including nucleotides and proteins. In particular, mammalian APs exert their properties by primarily targeting LPS (a TLR4 agonist), flagellin (a TLR5 agonist) and CpG DNA (a TLR9 agonist). APs also degrade intestine luminal NTPs (e.g., ATP, GTP, etc.), which promote the growth of good bacteria and reverses dysbiosis. Accordingly, APs may find clinical use as, for example, microbiome preserving agents for treating various gastrointestinal (Gl) disorders.

Orally administered intestinal alkaline phosphatase (IAP) has previously been shown to ameliorate metabolic syndrome in mice. It has also been shown that in humans, fecal levels of IAP correlate with Type 2 diabetes, independently of obesity. WO2018/175413 discloses a modified-release formulation comprising an intestinal alkaline phosphatase (IAP) to treat a metabolic disease, such as obesity, diabetes and/or metabolic syndrome and envisages the administration of IAP to a subject undergoing fecal bacteriotherapy.

Furthermore, administration of certain commensal gut bacteria, such as *Bacteroides acidifaciens,* has been shown to prevent obesity and improve insulin sensitivity and overall metabolic syndrome in animal models.

Given the increasing rate of incidents of metabolic disorder due to high fat diets, there is a growing need for improved therapies and therapeutic compositions that treat and prevent metabolic disorders and diseases associated therewith.

### SUMMARY

The present invention provides an intestinal alkaline phosphatase (IAPs) constructs, including variants thereof, and a composition comprising at least one commensal gastrointestinal bacteria (or "gut bacteria") comprising *Bacteroides acidifaciens.* In some examples, the IAP construct is a mammalian IAP including, but not limited to, human IAP (hIAP), calf IAP (clAP), and bovine IAP (bIAP). In some examples, the IAP is secreted from the host cell. In various embodiments, the IAP and is administered orally.

In various examples, the present invention provides an additional therapeutic agent, such as compositions comprising specific commensal gut bacteria (e.g., *Bacteroides acidifaciens).* In some embodiments, a composition comprising *Bacteroides acidifaciens* is administered by fecal bacteriotherapy, such as fecal transplant. In other embodiments, the *Bacteroides acidifaciens* is administered orally.

In an aspect, the present invention provides a co-formulated IAP and composition comprising Bacteroides *acidifaciens.* In some embodiments, the co-formulation is administered to a patient simultaneously but the release of the additional therapeutic agent and the IAP from their respective dosage forms (or single unit dosage form if co-formulated) occurs sequentially.

Also described are methods for the therapeutic use of an IAP. In some aspects, an IAP is administered to a patient undergoing therapy with a composition comprising commensal gut bacteria, including, but not limited to, *Bacteroides acidifaciens.* For example, a patient is undergoing therapy with fecal microbiota transplantation (FMT). In other aspects, a therapeutically effective amount of a composition comprising commensal gut bacteria, including but not limited to, *Bacteroides acidifaciens,* is administered to a patient undergoing therapy with IAP. In various examples, the composition comprising *Bacteroides acidifaciens* is a fecal microbiota transplant. In other examples, the composition comprising *Bacteroides acidifaciens* is an isolated bacterial composition. In an example, described are methods for the treatment of a metabolic disorder. In certain examples, the present invention provides methods for the treatment of a metabolic disorder such as obesity, diabetes, and/or a metabolic syndrome. In various examples, described are methods for increasing or preserving the number of commensal bacteria and/or composition of the gut microbiome of the patient. In some examples, provided are methods for inhibiting the growth or decreasing the number of pathogenic bacteria in the gut microbiome of the patient.

### BRIEF DESCRIPTION OF THE DRAWINGS

**Figure 1** depicts sequences pertaining to alkaline phosphatase agents present in compositions and/or formulations described herein.
**Figure 2** depicts a stacked bar graph showing the frequency of gut bacteria at day 0 (pre-antibiotic treatment) and day 4 (after 3 days of treatment with streptomycin) at the genus level. The frequency of *Bacteroides acidifaciens,* specifically, is shown on the stacked bar graph and indicated by black lines.

### DETAILED DESCRIPTION

### Overview

The role of alkaline phosphatases (APs) in promoting growth of good bacteria and reversing dysbiosis is a significant and growing field of study in the advancement of treatment options for metabolic disorders such as metabolic syndrome, obesity, and diabetes (Type 1 or Type 2).

In particular, intestinal alkaline phosphatase (IAP) is an endogenous protein expressed by the intestinal epithelium that can be used to mitigate inflammation and maintain gut homeostasis. For example, loss of IAP expression or function is associated with increased intestinal inflammation, dysbiosis, bacterial translocation, and systemic inflammation. Its primary functions, among others, in maintaining intestinal homeostasis are generally recognized as the regulation of bicarbonate secretion and duodenal surface pH, long chain fatty acid absorption, mitigation of intestinal inflammation through detoxification of pathogen-associated molecular patterns, and regulation of the gut microbiome. Several substrates that are acted on by IAP's phosphatase functions include lipopolysaccharide (LPS), flagellin, CpG DNA, and nucleotide di- and tri-phosphates. Specifically, IAP is a target for therapeutics due to its ability to downregulate inflammation, regulate the microbiome, tighten the gut barrier through enhanced expression of claudins and occludins, and affect metabolism of adenosine tri-phosphate and diphosphate (ATP and ADP).

Certain commensal gut bacteria are known to modulate metabolites and influence host immunity, thereby playing a significant part in modulating metabolism and disorders associated therewith. For example, *Bacteroides acidifaciens* is a commensal gut bacteria that has been shown to prevent obesity and improve insulin sensitivity in mice.

Described herein are pharmaceutical compositions, formulations, and uses of a combination of one or more intestinal alkaline phosphatases (IAPs) and a composition comprising commensal gut bacteria, including *Bacteroides acidifaciens.* In certain aspects, the patient is undergoing therapy with a composition comprising commensal gut bacteria that includes *Bacteroides acidifaciens.*

### Alkaline Phosphatases (APs)

Described herein are pharmaceutical compositions, formulations, and uses of one or more alkaline phosphatases. Alkaline phosphatases are dimeric metalloenzymes that catalyze the hydrolysis of phosphate esters and dephosphorylate a variety of target substrates at physiological and higher pHs. Illustrative APs include intestinal alkaline phosphatase (IAP; e.g., calf IAP or bovine IAP, chicken IAP, goat IAP), placental alkaline phosphatase (PLAP), placental-like alkaline phosphatase, germ cell alkaline phosphatase (GCAP), tissue non-specific alkaline phosphatase (TNAP; which is primarily found in the liver, kidney, and bone), bone alkaline phosphatase, liver alkaline phosphatase, kidney alkaline phosphatase, bacterial alkaline phosphatase, fungal alkaline phosphatase, shrimp alkaline phosphatase, modified IAP, recombinant IAP, or any polypeptide comprising alkaline phosphatase activity.

In various examples, described is the use of mammalian alkaline phosphatases including intestinal alkaline phosphatase (IAP), placental alkaline phosphatase (PLAP), germ cell alkaline phosphatase (GCAP), and the tissue non-specific alkaline phosphatase (TNAP).

### Intestinal Alkaline Phosphatase (IAP)

The alkaline phosphatase is IAP. IAP is produced in the proximal small intestine and is bound to the enterocytes via a glycosyl phosphatidylinositol (GPI) anchor. Some IAP is released into the intestinal lumen in conjunction with vesicles shed by the cells and as soluble protein stripped from the cells via phospholipases. The enzyme then traverses the small and large intestine such that some active enzyme can be detected in the feces. In an example, the IAP is human IAP (hIAP). In an example, the IAP is calf IAP (clAP), also known as bovine IAP (bIAP). There are multiple isozymes of bIAP, for example, with bIAP II and IV having higher specific activity than bIAP I. In an embodiment, the IAP is any one of the clAP or bIAP isozymes (e.g., bIAP I, II, and IV). In an embodiment, the IAP is bIAP II. In another embodiment, the IAP is bIAP IV.

In various examples, the IAP of the present invention has greater specific enzymatic activity than commercially-available APs, e.g., calf IAP (clAP).

### IAP variants

Also included within the definition of IAPs are IAP variants. An IAP variant has at least one or more amino acid modifications, generally amino acid substitutions, as compared to the parental wild-type sequence. In some examples, an IAP comprises an amino sequence having at least about 60% (e.g. about 60%, or about 61%, or about 62%, or about 63%, or about 64%, or about 65%, or about 66%, or about 67%, or about 68%, or about 69%, or about 70%, or about 71%, or about 72%, or about 73%, or about 74%, or about 75%, or about 76%, or about 77%, or about 78%, or about 79%, or about 80%, or about 81%, or about 82%, or about 83%, or about 84%, or about 85%, or about 86%, or about 87%, or about 88%, or about 89%, or about 90%, or about 91%, or about 92%, or about 93%, or about 94%, or about 95%, or about 96%, or about 97%, or about 98%, or about 99%) sequence identity with any of the sequences disclosed herein. In addition, IAP variants retain most or all of their biochemical activity, measured as described herein.

### GPI anchored proteins

Mammalian alkaline phosphatases are GPI anchored proteins. They have signal peptides and are translated into the secretory pathway. Once in the endoplasmic reticulum (ER), the proteins are glycosylated and folded. There are two disulfide bonds as well as a single free cysteine that is apparently not accessible on the surface. In the late ER, the carboxy terminus is removed and the GPI anchor is appended. GPI anchoring is therefore a process that occurs at the carboxy terminus of the alkaline phosphatase. The inclusion of stop codons at the anchor site enables secretion of biologically active protein (presumably the homodimer). While there is no consensus sequence, the carboxy terminus includes three amino acids, termed omega, omega +1, and omega +2 which are followed by a short stretch of hydrophilic amino acids and then a stretch of hydrophobic amino acids. Without wishing to be bound by theory, it is believed that the hydrophobicity is critical for embedding the carboxy terminus in the ER membrane. There, an enzymatic reaction replaces the carboxy terminus with the GPI anchor.

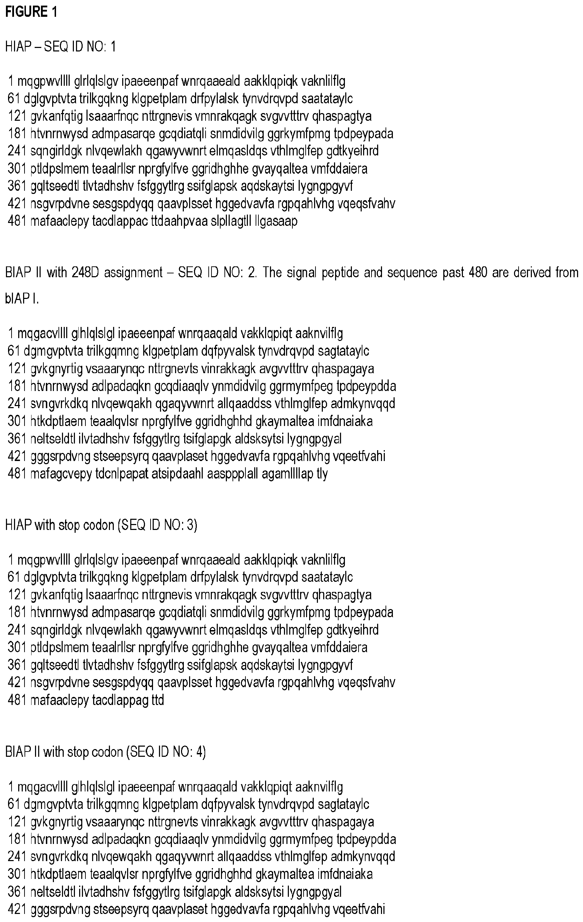

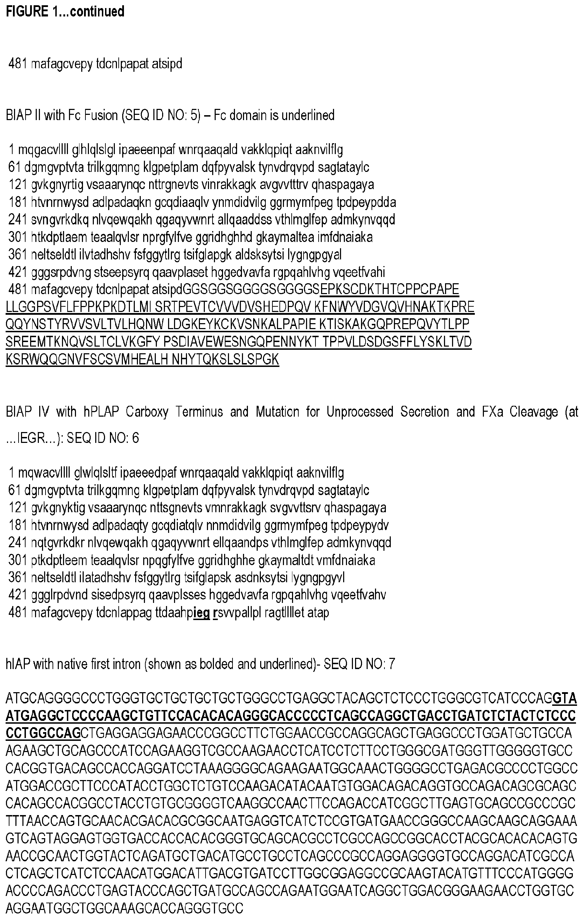

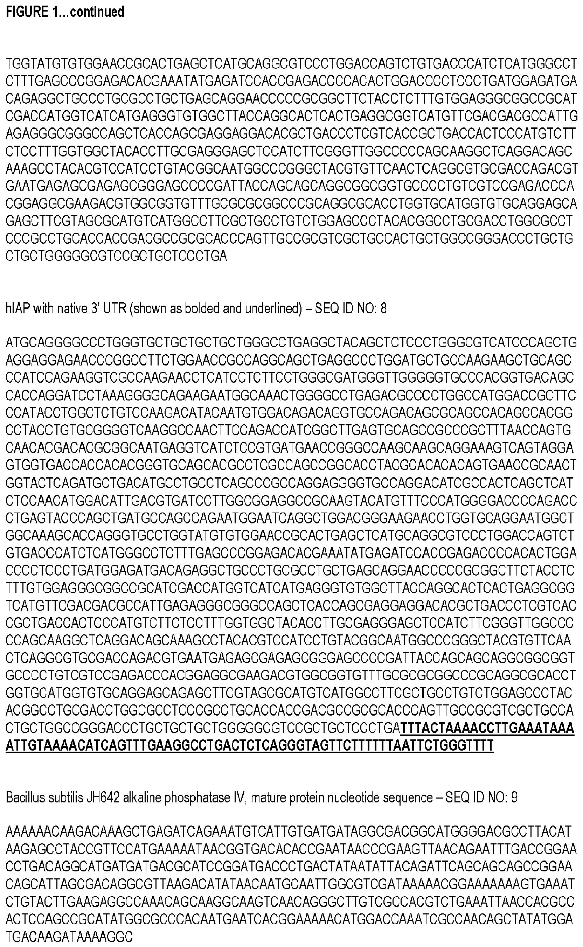

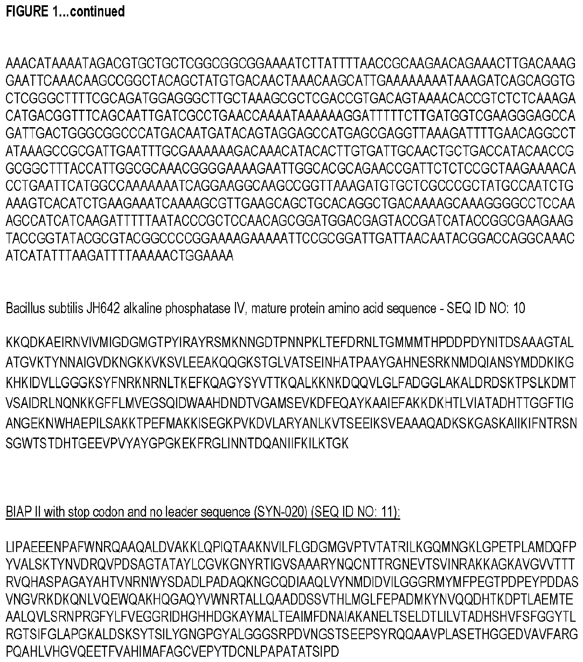

In other examples, the IAP of the invention is a secreted protein; that is, in some examples, the IAP is not GPI anchored, leading to secretion rather than intracellular retention. This can be accomplished in several ways. In some examples, the IAP may lack the GPI anchor site, e.g. have the DAAH site removed, leading to secretion. Alternatively, this can be accomplished in some examples, the IAP comprises a stop codon that is inserted immediately before the GPI anchor site. In an example, the IAP comprises a stop codon after the aspartate in the DAAH consensus site (e.g., at amino acid 503 of hIAP and bIAP IV or amino acid 506 of bIAP II). Figure 1 depicts HIAP with a stop codon (SEQ ID NO: 3) and bIAP II with a stop codon (SEQ ID NO: 4).

### Human IAP

In various examples, the IAP is human IAP (hIAP). In some examples, the IAP is hIAP comprising the amino acid sequence of SEQ ID NO: 1 as depicted in Figure 1 or a variant as described herein, as long as the hIAP variant retains at least 75, 80, 85, 90, 95, 96, 97, 98, 99 or 100% of the phosphatase activity as compared to the wild type enzyme using an assay as outlined herein.

Included within the definition of hIAP are amino acid modifications, with amino acid substitutions finding particular use in the present invention. For example, without wishing to be bound by theory, it is believed that a cysteine at the carboxy terminus of the AP-based agent (e.g., at position 500 of SEQ ID NO: 1) may interfere with protein folding. Accordingly, in some examples, the AP-based agent includes a mutation of the cysteine (e.g., at position 500 of SEQ ID NO: 1). In some examples, the cysteine is replaced with any amino acid, although glycine finds particular use in some examples. Furthermore, the C-terminal cysteine can also be deleted.

As will be appreciated by those in the art, additional amino acid modifications can be made in hIAP as discussed herein. For example, in some examples, a stop codon may be inserted after the aspartate in the DAAH consensus site (e.g., at amino acid 503 of hIAP). Figure 1 depicts hIAP with an inserted stop codon (SEQ ID NO: 3).

### Fusion Proteins

In various examples, provided are chimeric proteins. In some examples, provided are chimeric fusion proteins. For example, in various examples, provided is an isolated or recombinant alkaline phosphatase comprising a crown domain and a catalytic domain, wherein said crown domain and said catalytic domain are obtained from different alkaline phosphatases (e.g., human and bovine alkaline phosphatases). In other examples, the alkaline phosphatases are both human APs. In certain examples, provided are recombinant fusion proteins comprising human IAP and domains of human placental alkaline phosphatases. In certain examples, provided are chimeric hIAP-placenta fusion proteins.

In various examples, the AP-based agent of the invention is a fusion protein. In some examples, the AP-based agent comprises an alkaline phosphatase fused to a protein domain that replaces the GPI anchor sequence. In some examples, the alkaline phosphatase is fused to a protein domain that promotes protein folding and/or protein purification and/or protein dimerization and/or protein stability. In various examples, the AP-based agent fusion protein has an extended serum half-life.

In an examples, the alkaline phosphatase is fused to an immunoglobulin Fc domain and/or hinge region. In various examples, the immunoglobulin Fc domain and/or hinge region is derived from the Fc domain and/or hinge region of an antibody (e.g., of IgG, IgA, IgD, and IgE, inclusive of subclasses (e.g. IgG1, IgG2, IgG3, and IgG4, and IgA1 and IgA2)). In an example, the AP-based agent of the invention comprises an alkaline phosphatase fused to the hinge region and/or Fc domain of IgG.

In various examples, the AP-based agent of the invention is a pro-enzyme. In an example, the activity of the proenzyme is suppressed by a carboxy terminus. In an example, protease removal of the carboxy terminus reactivates the enzymatic activity of the alkaline phosphatase. In an example, the pro-enzyme is more efficiently secreted than the enzyme without the carboxy terminus.

In some examples, for generation of the pro-enzyme, the native carboxy terminus of the alkaline phosphatase is replaced with the analogous sequence from hPLAP. In some examples, a mutation is made in the hydrophobic carboxy tail to promote protein secretion without cleavage of the carboxy terminus. In an illustrative example, a single point mutation such as a substitution of leucine with e.g., arginine is generated in the hydrophobic carboxy terminus (e.g. allpllagtl is changed to e.g., allplragtl) to result in secretion of the enzyme without removal of the carboxy terminus.

### Bovine IAPs

In some embodiments, the IAP is bovine IAP (blAP).

### a. bIAP II

In various examples, the IAP is bovine IAP II (bIAP II) or a variant as described herein, as long as the bIAP variant retains at least 75, 80, 85, 90, 95, 96, 97, 98, 99 or 100% of the phosphatase activity using an assay as outlined herein. In an example, the bIAP II comprises the signal peptide and carboxy terminus of bIAP I. In an example, the bIAP II comprises an aspartate at position 248 (similar to bIAP IV). In an example, the bIAP II comprises the amino acid sequence of SEQ ID NO: 2. Figure 1 depicts BIAP II with 248D assignment - SEQ ID NO: 2. The signal peptide and sequence past 480 are derived from bIAP I.

Also included within the definition of bIAP II are amino acid variants as described herein. For example, in some embodiments, a stop codon may be inserted after the aspartate in the DAAH consensus site (e.g., at amino acid 506 of bIAP II). Figure 1 depicts bIAP II with an inserted stop codon (SEQ ID NO: 4).

In various embodiments, the bIAP II comprises the amino acid sequence of SEQ ID NO: 11.

### BIAP II with stop codon and no leader sequence (SYN-020) (SEQ ID NO: 11):

LIPAEEENPAFWNRQAAQALDVAKKLQPIQTAAKNVILFLGDGMGVPTVTATRILKGQMNGKL
GPETPLAMDQFPYVALSKTYNVDRQVPDSAGTATAYLCGVKGNYRTIGVSAAARYNQCNTTR
GNEVTSVINRAKKAGKAVGVVTTTRVQHASPAGAYAHTVNRNWYSDADLPADAQKNGCQDI
AAQLVYNMDIDVILGGGRMYMFPEGTPDPEYPDDASVNGVRKDKQNLVQEWQAKHQGAQY
VWNRTALLQAADDSSVTHLMGLFEPADMKYNVQQDHTKDPTLAEMTEAALQVLSRNPRGFY
LFVEGGRIDHGHHDGKAYMALTEAIMFDNAIAKANELTSELDTLILVTADHSHVFSFGGYTLRG
TSIFGLAPGKALDSKSYTSILYGNGPGYALGGGSRPDVNGSTSEEPSYRQQAAVPLASETHG
GEDVAVFARGPQAHLVHGVQEETFVAHIMAFAGCVEPYTDCNLPAPATATSIPD.

### Expression Variants

In various examples, the IAP is efficiently expressed and secreted from a host cell. In an example, the IAP is efficiently transcribed in a host cell. In another example, the IAP exhibits enhanced RNA stability and/or transport in a host cell. In another example, the IAP is efficiently translated in a host cell. In another example, the IAP exhibits enhanced protein stability.

In various examples, the IAPs are efficiently expressed in a host cell. In an example, the Kozak sequence of the DNA construct encoding the AP-based agent is optimized. The Kozak sequence is the nucleotide sequence flanking the ATG start codon that instructs the ribosome to start translation. There is flexibility in the design of a Kozak sequence, but one canonical sequence is GCCGCCACCATGG. The purine in the -3 position and the G in the +4 position are the most important bases for translation initiation. For hIAP, bIAP II, and bIAP IV, the second amino acid, that is, the one after the initiator methionine, is glutamine. Codons for glutamine all have a C in the first position. Thus, their Kozak sequences all have an ATGC sequence. Accordingly, in various examples, the ATGC sequence is changed to ATGG. This can be achieved by changing the second amino acid to a glycine, alanine, valine, aspartate, or glutamic acid, all of whose codons have a G in the first position. These amino acids may be compatible with signal peptide function. In alternative examples, the entire signal peptide is substituted for peptide having a canonical Kozak sequence and is derived from a highly expressed protein such as an immunoglobulin.

In various examples, the signal peptide of the IAP may be deleted and/or substituted. For example, the signal peptide may be deleted, mutated, and/or substituted (e.g., with another signal peptide) to ensure optimal protein expression.

In some examples, the DNA construct encoding the IAP comprises untranslated DNA sequences. Such sequences include an intron, which may be heterologous to the IAP protein or native to the IAP protein including the native first and/or second intron and/or a native 3' UTR. Without wishing to be bound by theory, it is believed that inclusion of these sequences enhances protein expression by stabilizing the mRNA. Accordingly, in various examples, the DNA construct encoding the IAP comprises the 5'UTR and/or the 3'UTR. Provided in Figure 1 are illustrative IAP DNA sequences with a first intron and a 3'UTR, including hIAP with native first intron (shown as bolded and underlined) - SEQ ID NO: 7; and hIAP with native 3' UTR (shown as bolded and underlined) - SEQ ID NO: 8.

In various examples, the IAP comprises a nucleotide sequence having at least about 60% (e.g. about 60%, or about 61%, or about 62%, or about 63%, or about 64%, or about 65%, or about 66%, or about 67%, or about 68%, or about 69%, or about 70%, or about 71%, or about 72%, or about 73%, or about 74%, or about 75%, or about 76%, or about 77%, or about 78%, or about 79%, or about 80%, or about 81%, or about 82%, or about 83%, or about 84%, or about 85%, or about 86%, or about 87%, or about 88%, or about 89%, or about 90%, or about 91%, or about 92%, or about 93%, or about 94%, or about 95%, or about 96%, or about 97%, or about 98%, or about 99%) sequence identity with any of the sequences disclosed herein.

In various examples, the IAP may comprise an amino acid sequence having one or more amino acid mutations relative to any of the protein sequences described herein. In some examples, the one or more amino acid mutations may be independently selected from substitutions, insertions, deletions, and truncations.

In various examples, the substitutions may also include non-classical amino acids (e.g. selenocysteine, pyrrolysine, N-formylmethionine β-alanine, GABA and δ-Aminolevulinic acid, 4-aminobenzoic acid (PABA), D-isomers of the common amino acids, 2,4-diaminobutyric acid, α-amino isobutyric acid, 4-aminobutyric acid, Abu, 2-amino butyric acid, γ-Abu, ε-Ahx, 6-amino hexanoic acid, Aib, 2-amino isobutyric acid, 3-amino propionic acid, ornithine, norleucine, norvaline, hydroxyproline, sarcosme, citrulline, homocitrulline, cysteic acid, t-butylglycine, t-butylalanine, phenylglycine, cyclohexylalanine, β-alanine, fluoro-amino acids, designer amino acids such as β-methyl amino acids, C α-methyl amino acids, N α-methyl amino acids, and amino acid analogs in general).

Mutations may be made to the IAP to select for agents with desired characteristics. For example, mutations may be made to generate IAPs with enhanced catalytic activity or protein stability. In various examples, directed evolution may be utilized to generate IAPs of the invention. For example, error-prone PCR and DNA shuffling may be used to identify mutations in the bacterial alkaline phosphatases that confer enhanced activity.

### Commensal Gut Bacteria

Described herein are pharmaceutical compositions, formulations, and uses of commensal gut bacteria. High densities of non-pathogenic bacteria, otherwise known as commensal bacteria, inhabit the gastrointestinal system, specifically the lower small intestine and colon. Distinct differences exist between the mucosal immune response to pathogens and commensals. In fact, certain commensal gut bacteria have been shown to influence and promote immunological tolerance and gut homeostasis. Specific commensals, such as, *Bacteroides acidifaciens,* have been shown to prevent obesity and improve insulin sensitivity and overall metabolic syndrome in animal models.

Described herein are pharmaceutical compositions, formulations, and uses of commensal gut bacteria. Such bacteria include *Bacteroides acidifaciens.* Other commensal bacteria can include, but are not limited to, bacterial members of the genus *Bacteroides,* anaerobic gram-positive cocci, such as *Peptostreptococcus sp., Clostridiales sp., Eubacterium sp., Lactobacillus sp., Clostridium sp,* members of phila *Firmicutes,* and members of phila *Proteobacteria.* In certain examples, the present invention contemplates pharmaceutical compositions, formulations, and uses of compositions comprising a variety of bacterial strains.

Described herein are pharmaceutical compositions, formulations, and uses of the commensal bacteria *Bacteroides acidifaciens.* For example, a patient is undergoing therapy with a composition comprising *Bacteroides acidifaciens.* In embodiments, the composition comprising *Bacteroides acidifaciens* is co-administered with IAP. In additional embodiments, the composition comprising *Bacteroides acidifaciens* is co-formulated with IAP.

### Fecal Microbiota Transplant (FMT)

In some embodiments, provided is administration of fecal microbiota transplant (FMT), and in some examples, the FMT occurs in conjunction with administration of IAP to a patient in need thereof. In various examples, the FMT comprises at least one commensal gut bacterial strain. For example, the FMT comprises *Bacteroides acidifaciens.* In various examples, the composition comprising *Bacteroides acidifaciens* is a fecal microbiota transplant (FMT). Without wishing to be bound by theory, it is posited that FMT can repopulate a patient's microbiome with diverse microorganisms to a patient in need thereof by taking material containing said microorganisms from a donor and transplanting those materials to said patient. In some embodiments, the fecal transplant comprises human stool or derivaties thereof. In such embodiments, the human stool or derivates are obtained from a human donor. In some examples, the fecal transplant comprises a synthetic material containing bacteria that are isolated from a human donor's gastrointestinal tract or other environments and grown in pure or mixed cultures. In various examples, the FMT is formulated as a pill or tablet.

The fecal transplant can be administered in a variety of ways, including, but not limited to, oral administration, colonoscopy, sigmoidoscopy, enema, naso-gastric intubation, naso-duodenal intubation, and naso-jejunal intubation.

### Isolated Bacterial Compositions

In various embodiments, the composition comprising at least one commensal gut bacteria, including *Bacteroides acidifaciens,* is an isolated bacterial composition. Specifically, the bacteria may have been isolated from human stool, the human GI tract, or other environments and grown in pure or mixed cultures. In various embodiments, the bacteria are isolated from a human donor. For example, bacteria may be isolated from a first subject (e.g., a first human subject), and formulated as described herein, and administered to a second subject (e.g., a second human subject) as described herein. Following isolation (techniques for which are apparent to one skilled in the art), the isolated bacteria can be formulated according to a desired administration route. Exemplary formulations of isolated bacterial compositions can include, but are not limited to, tablets, powders, capsules, lyophilized compositions, and aqueous formulations. In an example, the isolated bacterial composition is formulated in a capsule comprising *Bacteroides acidifaciens* in an aqueous phase.

Described herein is a composition comprising a consortia of bacteria (e.g., a multitude of bacterial strains). In some examples, the composition comprises a defined amount of bacterial strains, including but not limited to, the variety of commensal gut bacteria previously mentioned. Accordingly, the composition can comprise from 1-10, from 1-20, from 1-30, from 1-40, from 1-50, from 1-60, from 1-70, from 1-80, from 1-90, or from 1-100 commensal gut bacterial strains.

In some examples, the bacteria are viable. The term "viable," as used herein, generally refers to the ability of an organism, such as bacteria, to survive under particular conditions. In some examples, the term "viable" relates to the ability of an organism, such as bacteria, to survive upon administration and/or delivery and to retain metabolic activity once released into the target region.

### Methods of Making IAP

The IAPs are made using standard molecular biology techniques. For example, nucleic acid compositions encoding the IAPs of the invention are also provided, as well as expression vectors containing the nucleic acids and host cells transformed with the nucleic acid and/or expression vector compositions. As will be appreciated by those in the art, the protein sequences depicted herein can be encoded by any number of possible nucleic acid sequences, due to the degeneracy of the genetic code.

As is known in the art, the nucleic acids encoding the components of the invention can be incorporated into expression vectors as is known in the art, and depending on the host cells, used to produce the IAP compositions of the invention. Generally, the nucleic acids are operably linked to any number of regulatory elements (promoters, origin of replication, selectable markers, ribosomal binding sites, inducers, etc.). The expression vectors can be extra-chromosomal or integrating vectors.

The nucleic acids and/or expression vectors of the invention are then transformed into any number of different types of host cells as is well known in the art, including mammalian, bacterial, yeast, insect and/or fungal cells, with mammalian cells (e.g. CHO cells), finding use in many embodiments.

The IAPs are made by culturing host cells comprising the expression vector(s) as is well known in the art. Once produced, traditional purification steps are done.

### Formulations

The present invention provides the described IAP and composition comprising *Bacteroides acidifaciens* (and/or additional therapeutic agents) in various formulations. Any IAP and/or or composition comprising Bacteroides *acidifaciens* (and/or additional therapeutic agents) described herein can take the form of tablets, pills, pellets, capsules, capsules containing liquids, capsules containing multiparticulates, powders, solutions, emulsion, drops, suppositories, emulsions, aerosols, sprays, suspensions, delayed-release formulations, sustained-release formulations, controlled-release formulations, or any other form suitable for use.

The formulations comprising the IAP and composition comprising *Bacteroides acidifaciens* (and additional therapeutic agents) may conveniently be presented in unit dosage forms. For example, the dosage forms may be prepared by methods which include the step of bringing the therapeutic agents into association with a carrier, which constitutes one or more accessory ingredients. For example, the formulations are prepared by uniformly and intimately bringing the therapeutic agent into association with a liquid carrier, a finely divided solid carrier, or both, and then, if necessary, shaping the product into dosage forms of the desired formulation (e.g., wet or dry granulation, powder blends, etc., followed by press tableting).

In various embodiments, the IAP and/or composition comprising *Bacteroides acidifaciens* (and additional therapeutic agents) described herein are formulated as compositions adapted for a mode of administration described herein.

In some embodiments, the IAP and the composition comprising *Bacteroides acidifaciens* are co-formulated.

In various examples, the formulation comprising IAP is resistant to compression and therefore suitable for tableting. In various examples, the formulation comprising *Bacteroides acidifaciens* is resistant to compression and therefore suitable for tableting. The IAP can be provided in a powder form that is then tableted, e.g., by physical compression of dried materials.

In various examples, the IAP and composition comprising *Bacteroides acidifaciens* is stable and/or active in the GI tract, e.g. in one or more of the mouth, esophagus, stomach, duodenum, small intestine, duodenum, jejunum, ileum, large intestine, colon transversum, colon descendens, colon ascendens, colon sigmoidenum, cecum, and rectum. In a specific example, the IAP and composition comprising *Bacteroides acidifaciens* is stable in the large intestine, optionally selected from one or more of colon transversum, colon descendens, colon ascendens, colon sigmoidenum and cecum. In a specific example, the IAP and composition comprising *Bacteroides acidifaciens* is stable in the small intestine, optionally selected from one or more of duodenum, jejunum, and ileum. In some examples, the IAP and composition comprising *Bacteroides acidifaciens* is resistant to proteases in the GI tract, including for example, the small intestine. In some examples, the IAP and composition comprising Bacteroides *acidifaciens* is substantially active at a pH of about 5.0 or above. For example, the IAP and/or composition comprising *Bacteroides acidifaciens* may be substantially active at a pH of about 6.0 to about 12, e.g. about 6.0, or about 6.1, or about 6.2, or about 6.3, or about 6.4, or about 6.5, or about 6.6, or about 6.7, or about 6.8, or about 6.9, or about 7.0, or about 7.1, or about 7.2, or about 7.3, or about 7.4, or about 7.5, or about 8.0, or about 8.5, or about 9.0, or about 9.5, or about 10.0, or about 10.5, or about 11.0, or about 11.5, or about 12.0 (including, for example, via formulation, as described herein). In some examples, stable refers to an enzyme that has a long enough half-life and maintains sufficient activity for therapeutic effectiveness.

In various examples, the IAP and composition comprising *Bacteroides acidifaciens* is stable in chyme. In order to assess IAP and composition comprising *Bacteroides acidifaciens* stability in chyme, samples of IAPs and compositions comprising *Bacteroides acidifaciens* are incubated in human chyme at 37°C. Stability is then evaluated by assessing aliquots withdrawn from the incubated samples at 0, 0.5, 1, 2, 3, 4, 5, and 6 hours for AP activity using a para-nitrophenyl phosphate (pNPP) AP substrate. Different chyme specimens can be used for evaluation of stability, including mixed chyme samples. Chyme samples are characterized for pH, liquid content, and protease activity.

In some examples, the IAP described herein includes derivatives that are modified, i.e., by the covalent attachment of any type of molecule to the alkaline phosphatase such that covalent attachment does not prevent the activity of the enzyme. For example, derivatives include alkaline phosphatases that have been modified by, inter alia, glycosylation, lipidation, acetylation, pegylation, phosphorylation, amidation, derivatization by known protecting/blocking groups, proteolytic cleavage, linkage to a cellular ligand or other protein, etc. Any of numerous chemical modifications can be carried out, including, but not limited to specific chemical cleavage, acetylation, formylation, metabolic synthesis of tunicamycin, etc. Additionally, the derivative can contain one or more non-classical amino acids. In various examples, the IAP is glycosylated to ensure proper protein folding.

### Pharmaceutically acceptable salts

The IAP and composition comprising *Bacteroides acidifaciens* described herein can possess a sufficiently basic functional group, which can react with an inorganic or organic acid, or a carboxyl group, which can react with an inorganic or organic base, to form a pharmaceutically acceptable salt. A pharmaceutically acceptable acid addition salt is formed from a pharmaceutically acceptable acid, as is well known in the art. Such salts include the pharmaceutically acceptable salts listed in, for example, Journal of Pharmaceutical Science, 66, 2-19 (1977) and The Handbook of Pharmaceutical Salts; Properties, Selection, and Use. P. H. Stahl and C. G. Wermuth (eds.), Verlag, Zurich (Switzerland) 2002.

The term "pharmaceutically acceptable salt" also refers to a salt of the alkaline phosphatases having an acidic functional group, such as a carboxylic acid functional group, and a base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or tri-alkylamines, dicyclohexylamine; tributyl amine; pyridine; N-methyl, N-ethylamine; diethylamine; triethylamine; mono-, bis-, or tris-(2-OH-lower alkylamines), such as mono-; bis-, or tris-(2-hydroxyethyl)amine, 2-hydroxy-tert-butylamine, or tris-(hydroxymethyl)methylamine, N,N-di-lower alkyl-N-(hydroxyl-lower alkyl)-amines, such as N,N-dimethyl-N-(2-hydroxyethyl)amine or tri-(2-hydroxyethyl)amine; N-methyl-D-glucamine; and amino acids such as arginine, lysine, and the like.

In some examples, the compositions described herein are in the form of pharmaceutically acceptable salts. In various examples, the formulation comprises 5%, about 10%, about 15%, about 20%, about 25%, about 30%, about 31%, about 32%, about 33%, about 34%, about 35%, about 36%, about 37%, about 38%, about 39%, about 40%, about 41%, about 42%, about 43%, about 43%, about 44%, about 45%, about 46%, about 47%, about 48%, about 49%, or about 50% by weight pharmaceutically acceptable salts.

### Pharmaceutical excipients

Further, any IAP and composition comprising *Bacteroides acidifaciens* described herein can be administered to a subject as a component of a composition that comprises a pharmaceutically acceptable carrier or vehicle. Such compositions can optionally comprise a suitable amount of a pharmaceutically acceptable excipient so as to provide the form for proper administration.

Pharmaceutical excipients can be liquids, such as water and oils, including those of petroleum, animal, vegetable, or synthetic origin, such as peanut oil, soybean oil, mineral oil, sesame oil and the like. The pharmaceutical excipients can be, for example, saline, gum acacia, gelatin, starch paste, talc, keratin, colloidal silica, urea and the like. In addition, auxiliary, stabilizing, thickening, lubricating, and coloring agents can be used. In one example, the pharmaceutically acceptable excipients are sterile when administered to a subject. Water is a useful excipient when any agent described herein is administered intravenously. Saline solutions and aqueous dextrose and glycerol solutions can also be employed as liquid excipients, specifically for injectable solutions. Suitable pharmaceutical excipients also include starch, glucose, cellulose, hypromellose, lactose, sucrose, trehalose, malt, rice, flour, chalk, silica gel, sodium stearate, glycerol monostearate, talc, sodium chloride, dried skim milk, glycerol, propylene, glycol, povidone, crosspovidone, water, ethanol and the like. Any agent described herein, if desired, can also comprise minor amounts of wetting or emulsifying agents, or pH buffering agents. Other examples of suitable pharmaceutical excipients are described in Remington's Pharmaceutical Sciences 1447-1676 (Alfonso R. Gennaro eds., 19th ed. 1995).

A suitable pharmaceutical excipient for the purposes of tableting can be Ludipress (Lactose, povidone, crospovidone; CAS-No.: 5989-81-1 + 9003-39-8).

Where necessary, the IAP and composition comprising *Bacteroides acidifaciens* and/or pharmaceutical compositions (and additional therapeutic agents) can include a solubilizing agent. Also, the agents can be delivered with a suitable vehicle or delivery device. Combination therapies outlined herein can be co-delivered in a single delivery vehicle or delivery device.

In one embodiment, the IAP and composition comprising *Bacteroides acidifaciens* described herein are formulated as compositions adapted for oral administration. Compositions for oral delivery can be in the form of tablets, lozenges, aqueous or oily suspensions, granules, powders, sprinkles, emulsions, capsules, syrups, or elixirs, for example. Orally administered compositions can comprise one or more agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, where in tablet or pill form, the compositions can be coated to delay disintegration to provide a sustained action over an extended period of time. Selectively permeable membranes surrounding an osmotically active agent driving any IAP (and/or additional therapeutic agents) described herein are also suitable for orally administered compositions. In these latter platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time-delay material such as glycerol monostearate or glycerol stearate can also be useful. Oral compositions can include excipients such as mannitol, lactose, starch, magnesium stearate, sodium saccharin, cellulose, ethacrylic acid and derivative polymers thereof, and magnesium carbonate. In one example, the excipients are of pharmaceutical grade. Suspensions, in addition to the active compounds, may contain suspending agents such as, for example, ethoxylated isostearyl alcohols, polyoxyethylene sorbitol and sorbitan esters, microcrystalline cellulose, aluminum metahydroxide, bentonite, agar-agar, tragacanth, etc., and mixtures thereof.

In various examples, the IAP and/or composition comprising *Bacteroides acidifaciens* (and/or additional therapeutic agent) are formulated as solid dosage forms such as tablets, dispersible powders, granules, and capsules. In one example, the IAP and/or composition comprising *Bacteroides acidifaciens* (and/or additional therapeutic agent) are formulated as a capsule. In another example, the IAP and/or composition comprising *Bacteroides acidifaciens* (and/or additional therapeutic agent) are formulated as a tablet. In yet another example, the IAP and/or composition comprising *Bacteroides acidifaciens* (and/or additional therapeutic agent) are formulated as a soft-gel capsule. In a further example, the IAP and/or composition comprising Bacteroides *acidifaciens* (and/or additional therapeutic agent) are formulated as a gelatin capsule.

In various examples, the formulations of the IAP and/or composition comprising *Bacteroides acidifaciens* may additionally comprise a pharmaceutically acceptable carrier or excipient. As one skilled in the art will recognize, the formulations can be in any suitable form appropriate for the desired use and route of administration.

In some dosage forms, the agents described herein are mixed with at least one inert, pharmaceutically acceptable excipient or carrier such as sodium citrate, dicalcium phosphate, etc., and/or a) fillers or extenders such as starches, lactose, sucrose, glucose, mannitol, silicic acid, microcrystalline cellulose, and Bakers Special Sugar, etc., b) binders such as, for example, carboxymethylcellulose, alginates, gelatin, polyvinylpyrrolidone, sucrose, acacia, polyvinyl alcohol, polyvinylpyrrolidone, methylcellulose, hydroxypropyl cellulose (HPC), and hydroxymethyl cellulose etc., c) humectants such as glycerol, etc., d) disintegrating agents such as agar-agar, calcium carbonate, potato or tapioca starch, alginic acid, certain silicates, sodium carbonate, cross-linked polymers such as crospovidone (cross-linked polyvinylpyrrolidone), croscarmellose sodium (cross-linked sodium carboxymethylcellulose), sodium starch glycolate, etc., e) solution retarding agents such as paraffin, etc., f) absorption accelerators such as quaternary ammonium compounds, etc., g) wetting agents such as, for example, cetyl alcohol and glycerol monostearate, etc., h) absorbents such as kaolin and bentonite clay, etc., and i) lubricants such as talc, calcium stearate, magnesium stearate, solid polyethylene glycols, sodium lauryl sulfate, glyceryl behenate, etc., and mixtures of such excipients. One of skill in the art will recognize that particular excipients may have two or more functions in the oral dosage form. In the case of an oral dosage form, for example, a capsule or a tablet, the dosage form may also comprise buffering agents.

### Surface active agents

The formulation can additionally include a surface active agent. Surface active agents suitable for use in the present invention include, but are not limited to, any pharmaceutically acceptable, non-toxic surfactant. Classes of surfactants suitable for use in the compositions of the invention include, but are not limited to polyethoxylated fatty acids, PEG-fatty acid diesters, PEG-fatty acid mono- and di-ester mixtures, polyethylene glycol glycerol fatty acid esters, alcohol-oil transesterification products, polyglycerized fatty acids, propylene glycol fatty acid esters, mixtures of propylene glycol esters-glycerol esters, mono- and diglycerides, sterol and sterol derivatives, polyethylene glycol sorbitan fatty acid esters, polyethylene glycol alkyl ethers, sugar esters, polyethylene glycol alkyl phenols, polyoxyethylene-olyoxypropylene block copolymers, sorbitan fatty acid esters, lower alcohol fatty acid esters, ionic surfactants, and mixtures thereof. In some examples, compositions may comprise one or more surfactants including, but not limited to, sodium lauryl sulfate, polysorbate 20, polysorbate 40, polysorbate 60, polysorbate 80, and triethyl citrate.

The formulation can also contain pharmaceutically acceptable plasticizers to obtain the desired mechanical properties such as flexibility and hardness. Such plasticizers include, but are not limited to, triacetin, citric acid esters, triethyl citrate, phthalic acid esters, dibutyl sebacate, cetyl alcohol, polyethylene glycols, polysorbates or other plasticizers.

The formulation can also include one or more application solvents. Some of the more common solvents that can be used to apply, for example, a delayed-release coating composition include isopropyl alcohol, acetone, methylene chloride and the like.

The formulation can also include one or more alkaline materials. Alkaline material suitable for use in compositions of the invention include, but are not limited to, sodium, potassium, calcium, magnesium and aluminum salts of acids such as phosphoric acid, carbonic acid, citric acid and other aluminum/magnesium compounds. In addition, the alkaline material may be selected from antacid materials such as aluminum hydroxides, calcium hydroxides, magnesium hydroxides and magnesium oxide.

In various examples, the formulation can additionally include magnesium and/or zinc. Without wishing to be bound by theory, the inclusion of magnesium and/or zinc in the formulation promotes protein folding (e.g., dimer formation) and bioactivity of the IAP. In some examples, the formulation can include magnesium at a concentration of from about 1 µM to greater than 5 mM (e.g., from about 1 µM to more than 5 mM), inclusive of all ranges and values therebetween. In an example, the magnesium is present in the formulation at 1.0 mM. In some examples, the formulation can include zinc at a concentration of about 1 µM to greater than 1 mM (e.g., from about 1 µM to more than 1 mM), inclusive of all ranges and values therebetween. In an example, the zinc is present in the formulation at 0.1 mM. In various examples, the formulation of the present invention is substantially free of metal chelators.

In various examples, the pH of the formulation ensures that the IAP is properly folded (e.g., dimer formation) and is bioactive. In some examples, the formulation is maintained at a pH such that the amino acids which coordinate the binding of magnesium and/or zinc within the AP-based agent are not protonated. Protonation of such coordinating amino acids may lead to loss of metal ions and bioactivity and dimer disassociation. In various examples, the pH of the formulation is greater than about 6, about 6.5, about 7, about 7.5, about 8, about 8.5, about 9, about 9.5, about 10, about 10.5, about 11, about 11.5, or about 12.

Besides inert diluents, the oral compositions can also include adjuvants such as sweetening, flavoring, and perfuming agents.

### Delivery

Various methods may be used to formulate and/or deliver the agents described herein to a location of interest. For example, the IAP and/or composition comprising *Bacteroides acidifaciens* (and/or additional therapeutic agents) described herein may be formulated for delivery to the GI tract. The GI tract includes organs of the digestive system such as mouth, esophagus, stomach, duodenum, small intestine, large intestine and rectum and includes all subsections thereof (e.g. the small intestine may include the duodenum, jejunum and ileum; the large intestine may include the colon transversum, colon descendens, colon ascendens, colon sigmoidenum and cecum). For example, the IAP and/or composition comprising *Bacteroides acidifaciens* (and/or additional therapeutic agents) described herein may be formulated for delivery to one or more of the stomach, small intestine, large intestine and rectum and includes all subsections thereof (e.g. duodenum, jejunum and ileum, colon transversum, colon descendens, colon ascendens, colon sigmoidenum and cecum). In some examples, the compositions described herein may be formulated to deliver to the gut. In some examples, the compositions described herein may be formulated to deliver to the upper or lower GI tract. In an example, the IAP and/or composition comprising *Bacteroides acidifaciens* (and/or additional therapeutic agents) may be administered to a subject, by, for example, directly or indirectly contacting the mucosal tissues of the GI tract.

In various examples, the administration of the IAP and/or composition comprising *Bacteroides acidifaciens* (and/or additional therapeutic agents) is into the GI tract via, for example, oral delivery, nasogastral tube, intestinal intubation (e.g. an enteral tube or feeding tube such as, for example, a jejunal tube or gastro-jejunal tube, etc.), direct infusion (e.g., duodenal infusion), endoscopy, colonoscopy, sigmoidoscopy or enema.

For example, described herein are modified release formulations comprising at least one IAP (and/or additional therapeutic agents), wherein the formulation releases a substantial amount of the IAP (and/or additional therapeutic agents) into one or more regions of the GI tract. For example, the formulation may release at least about 60% of the AP-based agent after the stomach and into one or more regions of the GI tract.

In various examples, the modified-release formulation of the present invention releases at least 60% of the IAP (or additional therapeutic agents) after the stomach into one or more regions of the intestine. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the IAP (or additional therapeutic agents) in the intestines.

In various examples, the modified-release formulation of the present invention releases at least 60% of the IAP (or additional therapeutic agents) in the small intestine. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the AP-based agent (or additional therapeutic agents) in the small intestine (e.g., one or more of duodenum, jejunum, ileum, and ileocecal junction).

In various examples, the modified-release formulation of the present invention releases at least 60% of the IAP (or additional therapeutic agents) in the large intestine. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the AP-based agent (or additional therapeutic agents) in the large intestine (e.g., one or more of cecum, ascending, transverse, descending or sigmoid portions of the colon, and rectum).

In various examples, the modified-release formulation does not substantially release the AP-based agent (or additional therapeutic agents) in the stomach.

In certain examples, the modified-release formulation releases the IAP and/or composition comprising Bacteroides *acidifaciens* (or additional therapeutic agents) above a specific pH. For example, the modified-release formulation is substantially stable in an acidic environment and substantially unstable (e.g., dissolves rapidly or is physically unstable) in a near neutral to alkaline environment. In some examples, stability is indicative of not substantially releasing while instability is indicative of substantially releasing. For example, the modified-release formulation is substantially stable at a pH of about 7.0 or less, or about 6.5 or less, or about 6.0 or less, or about 5.5 or less, or about 5.0 or less, or about 4.5 or less, or about 4.0 or less, or about 3.5 or less, or about 3.0 or less, or about 2.5 or less, or about 2.0 or less, or about 1.5 or less, or about 1.0 or less. In some examples, the formulations are stable in lower pH areas and therefore do not substantially release in, for example, the stomach. In some examples, the modified-release formulation is substantially stable at a pH of about 1 to about 5 or lower and substantially unstable at pH values that are greater. In these examples, the modified-release formulation does not substantially release in the stomach. In these examples, the modified-release formulation substantially releases in the small intestine (e.g. one or more of the duodenum, jejunum, and ileum) and/or large intestine (e.g. one or more of the cecum, ascending colon, transverse colon, descending colon, and sigmoid colon). In some examples, modified-release formulation is substantially stable at a pH of about 4 to about 7 or lower and consequentially is substantially unstable at pH values that are greater and therefore is not substantially released in the stomach and/or proximal small intestine (e.g. one or more of the duodenum, jejunum). In these examples, the modified-release formulation substantially releases in the distal small intestine or large intestine (e.g. one or more of the cecum, ascending colon, transverse colon, descending colon, and sigmoid colon). In various examples, the pH values recited herein may be adjusted as known in the art to account for the state of the subject, e.g. whether in a fasting or postprandial state.

In some examples, the modified-release formulation is substantially stable in gastric fluid and substantially unstable in intestinal fluid and, accordingly, is substantially released in the small intestine (e.g. one or more of the duodenum, jejunum, and ileum) and/or large intestine (e.g. one or more of the cecum, ascending colon, transverse colon, descending colon, and sigmoid colon).

In some examples, the modified-release formulation is stable in gastric fluid or stable in acidic environments. These modified-release formulations release about 30% or less by weight of the alkaline phosphatase and/or additional therapeutic agent in the modified-release formulation in gastric fluid with a pH of about 4 to about 5 or less, or simulated gastric fluid with a pH of about 4 to about 5 or less, in about 15, or about 30, or about 45, or about 60, or about 90 minutes. Modified-release formulations may release from about 0% to about 30%, from about 0% to about 25%, from about 0% to about 20%, from about 0% to about 15%, from about 0% to about 10%, about 5% to about 30%, from about 5% to about 25%, from about 5% to about 20%, from about 5% to about 15%, from about 5% to about 10% by weight of the alkaline phosphatase and/or additional therapeutic agent in the modified-release formulation in gastric fluid with a pH of 4-5, or less or simulated gastric fluid with a pH of 4-5 or less, in about 15, or about 30, or about 45, or about 60, or about 90 minutes. Modified-release formulations of the invention may release about 1%, about 2%, about 3%, about 4%, about 5%, about 6%, about 7%, about 8%, about 9%, or about 10% by weight of the total alkaline phosphatase and/or additional therapeutic agent in the modified-release formulation in gastric fluid with a pH of 5 or less, or simulated gastric fluid with a pH of 5 or less, in about 15, or about 30, or about 45, or about 60, or about 90 minutes.

In some examples, the modified-release formulation is unstable in intestinal fluid. These modified-release formulations release about 70% or more by weight of the alkaline phosphatase and/or additional therapeutic agent in the modified-release formulation in intestinal fluid or simulated intestinal fluid in about 15, or about 30, or about 45, or about 60, or about 90 minutes. In some examples, the modified-release formulation is unstable in near neutral to alkaline environments. These modified-release formulations release about 70% or more by weight of the alkaline phosphatase and/or additional therapeutic agent in the modified-release formulation in intestinal fluid with a pH of about 4-5 or greater, or simulated intestinal fluid with a pH of about 4-5 or greater, in about 15, or about 30, or about 45, or about 60, or about 90 minutes. A modified-release formulation that is unstable in near neutral or alkaline environments may release 70% or more by weight of alkaline phosphatase and/or additional therapeutic agent in the modified-release formulation in a fluid having a pH greater than about 5 (e.g., a fluid having a pH of from about 5 to about 14, from about 6 to about 14, from about 7 to about 14, from about 8 to about 14, from about 9 to about 14, from about 10 to about 14, or from about 11 to about 14) in from about 5 minutes to about 90 minutes, or from about 10 minutes to about 90 minutes, or from about 15 minutes to about 90 minutes, or from about 20 minutes to about 90 minutes, or from about 25 minutes to about 90 minutes, or from about 30 minutes to about 90 minutes, or from about 5 minutes to about 60 minutes, or from about 10 minutes to about 60 minutes, or from about 15 minutes to about 60 minutes, or from about 20 minutes to about 60 minutes, or from about 25 minutes to about 90 minutes, or from about 30 minutes to about 60 minutes.

Examples of simulated gastric fluid and simulated intestinal fluid include, but are not limited to, those disclosed in the 2005 Pharmacopeia 23NF/28USP in Test Solutions at page 2858 and/or other simulated gastric fluids and simulated intestinal fluids known to those of skill in the art, for example, simulated gastric fluid and/or intestinal fluid prepared without enzymes.

In various examples, the modified-release formulation of the invention is substantially stable in chyme. For example, there is a loss of less than about 50% or about 40%, or about 30%, or about 20%, or about 10% of AP-based agent activity in about 10, or 9, or 8, or 7, or 6, or 5, or 4, or 3, or 2, or 1 hour from administration.

In various examples, the modified-release formulations are designed for immediate release (e.g. upon ingestion). In various examples, the modified-release formulations may have sustained-release profiles, i.e. slow release of the active ingredient(s) in the body (e.g., GI tract) over an extended period of time. In various examples, the modified-release formulations may have a delayed-release profile, i.e. not immediately release the active ingredient(s) upon ingestion; rather, postponement of the release of the active ingredient(s) until the composition is lower in the GI tract; for example, for release in the small intestine (e.g., one or more of duodenum, jejunum, ileum) or the large intestine (e.g., one or more of cecum, ascending, transverse, descending or sigmoid portions of the colon, and rectum). For example, a composition can be enteric-coated to delay release of the active ingredient(s) until it reaches the small intestine or large intestine.

### Enteric coating

In various examples, the formulations (e.g. IAP as a powder or tablet) are coated to provide protection of the active agent in the GI tract, including the stomach. For example, the formulations can be encapsulated in an enterically-coated capsule. Additionally, in some examples, the formulations (e.g. IAP as a powder or tablet) itself is coated with one or more coatings, e.g. one or more modified-release coatings as described herein (e.g. after a step of granulating the powder). Further, in some examples, the powder formulations (e.g. AP-based agent as a powder) can be compressed into a tablet that is enterically coated.

In various examples, the modified-release formulation may utilize one or more modified-release coatings such as delayed-release coatings to provide for effective, delayed yet substantial delivery of the alkaline phosphatase to the GI tract together with, optionally, additional therapeutic agents.

In various examples, the modified-release formulation may utilize one or more modified-release coatings such as delayed-release coatings to provide for effective, delayed yet substantial delivery of the IAP to the intestines together with, optionally, other additional therapeutic agents.

In one example, the delayed-release coating includes an enteric agent that is substantially stable in acidic environments and substantially unstable in near neutral to alkaline environments. In an example, the delayed-release coating contains an enteric agent that is substantially stable in gastric fluid. The enteric agent can be selected from, for example, solutions or dispersions of methacrylic acid copolymers, cellulose acetate phthalate, hydroxypropylmethyl cellulose phthalate, polyvinyl acetate phthalate, carboxymethylethylcellulose, and EUDRAGIT^{®}-type polymer (poly(methacrylic acid, methylmethacrylate), hydroxypropyl methylcellulose acetate succinate, cellulose acetate trimellitate, shellac or other suitable enteric coating polymers. The polymers are described in international pharmacopeias such as Ph.Eur., USP/NF, DMF, and JPE. The EUDRAGIT^{®}-type polymers include, for example, EUDRAGIT^{®} FS 30D, L 30 D-55, L 100-55, L 100, L 12,5, L 12,5 P, RL 30 D, RL PO, RL 100, RL 12,5, RS 30 D, RS PO, RS 100, RS 12,5, NE 30 D, NE 40 D, NM 30 D, S 100, S 12,5, and S 12,5 P. Similar polymers include Kollicoat^{®} MAE 30 DP and Kollicoat^{®} MAE 100 P. In some embodiments, one or more of EUDRAGIT^{®} FS 30D, L 30 D-55, L 100-55, L 100, L 12,5, L 12,5 P RL 30 D, RL PO, RL 100, RL 12,5, RS 30 D, RS PO, RS 100, RS 12,5, NE 30 D, NE 40 D, NM 30 D, S 100, S 12,5 S 12,5 P, Kollicoat^{®} MAE 30 DP and Kollicoat^{®} MAE 100 P is used. In various examples, the enteric agent may be a combination of the foregoing solutions or dispersions. In an example, the delayed-release coating includes the enteric agent EUDRAGIT^{®} L 100.

By way of example, there are various EUDRAGIT formulations that dissolve at rising pH, with formulations that dissolve at pH >5.5 (EUDRAGIT L30 D-550), pH >6.0 (EUDRAGIT L12, 5), and pH >7.0 (EUDRAGIT FS 30D). Since the ileum has the highest pH in the small intestine, ranging from 7.3 to 7.8, the use of EUDRAGIT FS 30D as an enteric agent, may delay dissolution until the ileum thereby localizing the release of the AP-based agent to the ileum. However, the jejunum has a pH that can range from 6.6 to 7.4, therefore, various EUDRAGIT formulations can be used to target release to this segment of the intestine. The different types of EUDRAGIT can be combined with each other, or multiple different types of EUDRAGIT coatings can be combined to fine tune the dissolution profile to achieve targeted delivery to achieve optimal function. For example, EUDRAGIT L100, EUDRAGIT S100, and triethyl citrate may be mixed together at a ratio of, for example, about 72.7/18.2/9.1, to form a coating that substantially releases at a pH of greater than about 6.2. In another example, EUDRAGIT L100, EUDRAGIT S100, and triethyl citrate may be mixed together at a ratio of, for example, about 30/60.9/9, to form a coating that substantially releases at a pH of greater than about 6.7. In a further example, DuoCoat^{™} (Kuecept, Ltd.) may be used that uses two coatings of enteric polymers (like EUDRAGIT), an outer layer, and an inner layer of partially neutralized enteric polymer and a buffer agent. The DuoCoat^{™} technology allows more rapid release of the therapeutic agent initiated at the targeted pH compared to a single coating of the enteric polymer (Liu et al., 2010, European J. Pharmaceutics and Biopharmaceuticals 47:311). Release was demonstrated to be targeted to the ileum and/or ileoceacal junction in 10 healthy volunteers (Varum et al., 2013, European J. Pharmaceutics and Biopharmaceuticals 84:573).

In certain examples, one or more coating system additives are used with the enteric agent. For example, one or more PlasACRYLTM additives may be used as an anti-tacking agent coating additive. Illustrative PlasACRYLTM additives include, but are not limited to PlasACRYLTM HTP20 and PlasACRYLTM T20.

In another example, the delayed-release coating may degrade as a function of time when in aqueous solution without regard to the pH and/or presence of enzymes in the solution. Such a coating may comprise a water insoluble polymer. Its solubility in aqueous solution is therefore independent of the pH. The term "pH independent" as used herein means that the water permeability of the polymer and its ability to release pharmaceutical ingredients is not a function of pH and/or is only very slightly dependent on pH. Such coatings may be used to prepare, for example, sustained release formulations. Suitable water insoluble polymers include pharmaceutically acceptable non-toxic polymers that are substantially insoluble in aqueous media, e.g., water, independent of the pH of the solution. Suitable polymers include, but are not limited to, cellulose ethers, cellulose esters, or cellulose ether-esters, i.e., a cellulose derivative in which some of the hydroxy groups on the cellulose skeleton are substituted with alkyl groups and some are modified with alkanoyl groups. Examples include ethyl cellulose, acetyl cellulose, nitrocellulose, and the like. Other examples of insoluble polymers include, but are not limited to, lacquer, and acrylic and/or methacrylic ester polymers, polymers or copolymers of acrylate or methacrylate having a low quaternary ammonium content, or mixture thereof and the like. Other examples of insoluble polymers include EUDRAGIT RS^{®}, EUDRAGIT RL^{®}, and EUDRAGIT NE^{®}. Insoluble polymers useful in the present invention include polyvinyl esters, polyvinyl acetals, polyacrylic acid esters, butadiene styrene copolymers, and the like. In one example, colonic delivery is achieved by use of a slowly-eroding wax plug (e.g., various PEGS, including for example, PEG6000) or pectin. In an example, described is the use of a delayed-release coating that degrade as a function of time which comprises a swell layer comprising croscarmellos sodium and hydroxyproplycellulose. In such example, the formulation may further include an osmotic rupture coating that comprises ethylcellulose such as ethylcellulose dispersions.

Alternatively, the stability of the modified-release formulation can be enzyme-dependent. Delayed-release coatings that are enzyme dependent will be substantially stable in fluid that does not contain a particular enzyme and substantially unstable in fluid containing the enzyme. The delayed-release coating will essentially disintegrate or dissolve in fluid containing the appropriate enzyme. Enzyme-dependent control can be brought about, for example, by using materials which release the active ingredient only on exposure to enzymes in the intestine, such as galactomannans. Also, the stability of the modified-release formulation can be dependent on enzyme stability in the presence of a microbial enzyme present in the gut flora. For example, the delayed-release coating may be degraded by a microbial enzyme present in the gut flora. In an example, the delayed-release coating may be degraded by bacteria present in the small intestine. In another example, the delayed-release coating may be degraded by bacteria present in the large intestine.

In various examples, the modified release formulation is designed for release in the colon. Various colon-specific delivery approaches may be utilized. For example, the modified release formulation may be formulated using a colon-specific drug delivery system (CODES) as described for example, in Li et al., AAPS PharmSciTech (2002), 3(4): 1-9. Drug release in such a system is triggered by colonic microflora coupled with pH-sensitive polymer coatings. For example, the formulation may be designed as a core tablet with three layers of polymer. The first coating is an acid-soluble polymer (e.g., EUDRAGIT E), the outer coating is enteric, along with a hydroxypropyl methylcellulose barrier layer interposed in between. In another example, colon delivery may be achieved by formulating the alkaline phosphatase (and/or additional therapeutic agent) with specific polymers that degrade in the colon such as, for example, pectin. The pectin may be further gelled or crosslinked with a cation such as a zinc cation. In an example, the formulation is in the form of ionically crosslinked pectin beads which are further coated with a polymer (e.g., EUDRAGIT polymer). Additional colon specific formulations include, but are not limited to, pressure-controlled drug delivery systems (prepared with, for example, ethylcellulose) and osmotic controlled drug delivery systems (i.e., ORDS-CT).

Formulations for colon specific delivery of the IAP and/or composition comprising *Bacteroides acidifaciens* (and/or additional therapeutic agents), as described herein, may be evaluated using, for example, in vitro dissolution tests. For example, parallel dissolution studies in different buffers may be undertaken to characterize the behavior of the formulations at different pH levels. Alternatively, in vitro enzymatic tests may be carried out. For example, the formulations may be incubated in fermenters containing suitable medium for bacteria, and the amount of drug released at different time intervals is determined. Drug release studies can also be done in buffer medium containing enzymes or rat or guinea pig or rabbit cecal contents and the amount of drug released in a particular time is determined. In a further example, in vivo evaluations may be carried out using animal models such as dogs, guinea pigs, rats, and pigs. Further, clinical evaluation of colon specific drug delivery formulations may be evaluated by calculating drug delivery index (DDI) which considers the relative ratio of RCE (relative colonic tissue exposure to the drug) to RSC (relative amount of drug in blood i.e. that is relative systemic exposure to the drug). Higher drug DDI indicates better colon drug delivery. Absorption of drugs from the colon may be monitored by colonoscopy and intubation.

In various example, the present formulations provide for substantial uniform dissolution of the AP-based agent (and/or additional therapeutic agent) in the area of release in the GI tract. In an example, the present formulation minimizes patchy or heterogeneous release of the AP-based agent.

In various example, provided are modified-release formulations that release multiple doses of the AP-based agent, at different locations along the intestines, at different times, and/or at different pH. In an illustrative example, the modified-release formulation comprises a first dose of the AP-based agent and a second dose of the AP-based agent, wherein the first dose and the second dose are released at different locations along the intestines, at different times, and/or at different pH. For example, the first dose is released at the duodenum, and the second dose is released at the ileum. In another example, the first dose is released at the jejunum, and the second dose is released at the ileum. In other examples, the first dose is released at a location along the small intestine (e.g., the duodenum), while the second dose is released along the large intestine (e.g., the ascending colon). In various examples, the modified-release formulation may release at least one dose, at least two doses, at least three doses, at least four doses, at least five doses, at least six doses, at least seven doses, or at least eight doses of the AP-based agent at different locations along the intestines, at different times, and/or at different pH.

In various examples, the formulations take the form of those as described in one or more of US Patent Nos. 8,535,713 and 8,9117,77 and US Patent Publication Nos. 20120141585, 20120141531, 2006/001896, 2007/0292523, 2008/0020018, 2008/0113031, 2010/0203120, 2010/0255087, 2010/0297221, 2011/0052645, 2013/0243873, 2013/0330411, 2014/0017313, and 2014/0234418.

In various examples, the formulations take the form of those described in one or more of US Patent Nos. 4,196,564; 4,196,565; 4,247,006; 4,250,997; 4,268,265; 5,317,849; 6,572,892; 7,712,634; 8,074,835; 8,398,912; 8,440,224; 8,557,294; 8,646,591; 8,739,812; 8,810,259; 8,852,631; and 8,911,788 and US Patent Publication Nos. 2014/0302132; 2014/0227357; 20140088202; 20130287842; 2013/0295188; 2013/0307962; and 20130184290.

In various examples, the process of formulating the AP-based agent is sufficiently gentle such that the tertiary structure of the AP-based agent (e.g., dimeric structure) is substantially intact. In various examples, the process of formulating the AP-based agent includes a step of refolding the AP-based agent. In such examples, the step of refolding the AP-based agent may include the addition of magnesium and/or cyclodextrin.

In various examples, the modified-release formulation is a modified-release powder formulation.

In various examples, the modified-release formulation including AP-based agents described herein, and variants thereof, and/or additional therapeutic agents is administered orally.

Suitable dosage forms for oral use include, for example, solid dosage forms such as tablets, capsules, powders, and granules. In various examples, the modified-release formulation is in the form of powders. In some examples, the powdered formulations of the present invention can be added to food (e.g. juices, strained and/or pureed foods (e.g. fruits, vegetables), sauces, infant formulas, milk, etc.). In various examples, the modified-release formulation is packaged in the form of a sachet. In various examples, the modified-release formulation is in the form of tablets. In an example, the modified-release formulation is in the form of tablets comprising powders. In various examples, the modified-release formulation is in the form of capsules. In an example, the modified-release formulation is in the form of capsules comprising powders.

In various examples, the modified-release formulation is in the form of powders. In various examples, the powders are formed by spray drying and/or by spray-dried dispersion (SDD) technology. In some examples, the powders comprising AP-based agents are formed by dissolving AP-based agents and polymers in a solvent and then spray-drying the solution. The resulting powder comprises the AP-based agents dispersed within a solid polymeric matrix.

Various types of polymers may be used for the modified-release formulation described herein. In some examples, the polymer is an enteric polymer that is substantially stable in acidic environments and substantially unstable in near neutral to alkaline environments. In an example, the enteric polymer is substantially stable in gastric fluid.

Illustrative polymers include, but are not limited to, copovidone, polyvinyl caprolactam-polyvinyl acetate-polyethyleneglycol copolymer, poly(vinylpyrrolidinone) (PVP), hydroxypropylmethylcellulose or hypromellose (HPMC), hypromellose phthalate (HPMCP), hydroxypropylmethylcellulose or hypromellose acetate succinate (HPMCAS), methacrylate/methacrylic acid copolymer, and mixtures thereof. In an example, the polymer is HPMCAS. In various examples, the poymer is HPMCAS LF, LG, MF, MG, HF, or HG. In an example, the polymer is HPMCAS-LF.

### Buffers

Various types of solvents/buffers may be used for preparation of the powders described herein. In an example, the solvents/buffers are organic solvents/buffers. Illustrative solvents/buffers that may be used to dissolve the AP-based agent and polymer prior to spray-drying include, but are not limited to, ethanol, methanol, acetone, IPA, tetrahydrafuran, dichloromethane, and mixtures thereof. In various examples, the solvent used is water such as distilled DI water. In various examples, the buffer used is monosodium phosphate monohydrate.

In some examples, enzyme co-factors including zinc and magnesium are used. In an example, the enzyme co-factor zinc is used. In an example, the zinc is provided as zinc sulfate heptahydrate. In another example, the enzyme co-factor magnesium is used. In an example, the magnesium is provided as magnesium sulfate heptahydrate.

In some examples, the formulation includes a protein stabilizer such as trehalose, sucrose, lactose, mannitol, Tween 80, or polyvinyl alcohol. In an example, the stabilizer is sucrose. In an example, the stabilizer is lactose.

In some examples, surfactants may be included for the preparation of the powders described herein. The surfactants may be used as solubilizers or emulsifying agents. Illustrative surfactants include, but are not limited to, vitamin E polyethylene glycol succinate, sorbitan monostearate - 60/80, polysorbate 20, polysorbate 80, and polyoxyl 40 hydrogenated castor oil.

In various examples, the powders comprising AP-based agents becomes a gel. In various examples, the powders comprising an AP-based agent becomes a gel in the intestines. In various examples, the AP-based agent is released from the gel into one or more regions of the intestines. In various examples, at pH values greater than about 5 (e.g. about 5, or 6, or 7, or 8, or 9) the gel transforms back into the solution phase and releases the AP enzyme. In various examples, the gel is used to control the release of the AP-based agent in the intestines. In some examples, the AP-based agent is released from the gel into one or more of the group consisting of the small intestine, duodenum, jejunum, ileum, large intestine, colon transversum, colon descendens, colon ascendens, colon sigmoidenum, cecum, and rectum.

In various examples, the formulation described herein is in the form of powders comprising the AP-based agent dispersed within a solid polymeric matrix. In some examples, the powders are formed by dissolving AP-based agent and polymers in a solvent to form a solution that is subsequently spray-dried. In various examples, the solution for spray-drying comprises about 0.1-1% by weight of AP-based agent. For example, the AP-based agent may be present about 0.1%, about 0.15%, about 0.2%, about 0.25%, about 0.3%, about 0.35%, about 0.4%, about 0.45%, about 0.5%, about 0.55%, about 0.6%, about 0.65%, about 0.7%, about 0.75%, about 0.8%, about 0.85%, about 0.9%, about 0.95%, or about 1.0% by weight. In some examples, the solution comprises about 1-10% by weight a polymer (e.g., HPMCAS-LF). For example, the polymer may be present at about 1%, about 2%, about 3%, about 4%, about 5%, about 6%, about 7%, about 8%, about 9%, or about 10% by weight. In some examples, the solution comprises about 0.05-0.5% by weight buffer (e.g., monosodium phosphate monohydrate). For example, the buffer may be present at about 0.05%, about 0.06%, about 0.07%, about 0.08%, about 0.09%, about 0.10%, about 0.11%, about 0.12%, about 0.13%, about 0.14%, about 0.15%, about 0.16%, about 0.17%, about 0.18%, about 0.19%, about 0.20%, about 0.25%, about 0.30%, about 0.35%, about 0.40%, about 0.45%, or about 0.50% by weight. In some examples, the solution comprises about 0.001-0.01% by weight zinc (e.g., zinc sulfate heptahhydrate). For example, the zinc may be present at about 0.001%, about 0.002%, about 0.003%, about 0.004%, about 0.005%, about 0.006%, about 0.007%, about 0.008%, about 0.009%, or about 0.01% by weight. In some examples, the solution comprises about 0.01-0.1% by weight magnesium (e.g., magnesium sulfate heptahhydrate). For example, the magnesium may be present at about 0.01%, about 0.02%, about 0.03%, about 0.04%, about 0.05%, about 0.06%, about 0.07%, about 0.08%, about 0.09%, or about 0.1% by weight. In some examples, the solution comprises about 0.1-1% by weight a protein stabilizer (e.g., trehalose). For example, the protein stabilizer may be present at about 0.1%, about 0.2%, about 0.3%, about 0.4%, about 0.5%, about 0.6%, about 0.7%, about 0.8%, about 0.9%, or about 1% by weight. In some examples, the solution comprises about 90-99.9% by weight solvent (e.g., water). For example, the solvent may be present at about 90%, about 91%, about 92%, about 93%, about 94%, about 95%, about 96%, about 97%, about 98%, or about 99% by weight.

In various examples, the modified-release formulation is in the form of tablets or capsules. In some examples, the modified-release formulation is in the form of tablets or capsules comprising the powders described herein. A variety of approaches for generating tablets or capsules may be utilized to include powders described herein. In some examples, tablets are generated by granulation such as dry granulation. In such examples, the powders are pre-compressed and the resulting tablet or slug is milled to yield granules. Alternatively, the powders are pre-compressed with pressure rolls to yield granules. In yet other examples, the powders are encapsulated into capsules. In an example, the capsule is a gelatin capsule, such as a hard gelatin capsule. In another example, the capsule is a hydroxypropyl methylcellulose (HPMC) capsule.

In various examples, the tablets or capsules comprise a delayed-release coating that includes an enteric agent that is substantially stable in acidic environments and substantially unstable in near neutral to alkaline environments. In an example, the delayed-release coating contains an enteric agent that is substantially stable in gastric fluid. The enteric agent can be selected from, for example, solutions or dispersions of methacrylic acid copolymers, cellulose acetate phthalate, hydroxypropylmethyl cellulose phthalate, polyvinyl acetate phthalate, carboxymethylethylcellulose, and EUDRAGIT^{®}-type polymer (poly(methacrylic acid, methylmethacrylate), hydroxypropyl methylcellulose acetate succinate, cellulose acetate trimellitate, shellac or other suitable enteric coating polymers. The polymers are described in international pharmacopeias such as Ph.Eur., USP/NF, DMF, and JPE. The EUDRAGIT^{®}-type polymers include, for example, EUDRAGIT^{®} FS 30D, L 30 D-55, L 100-55, L 100, L 12,5, L 12,5 P, RL 30 D, RL PO, RL 100, RL 12,5, RS 30 D, RS PO, RS 100, RS 12,5, NE 30 D, NE 40 D, NM 30 D, S 100, S 12,5, and S 12,5 P. Similar polymers include Kollicoat^{®} MAE 30 DP and Kollicoat^{®} MAE 100 P. In some examples, one or more of EUDRAGIT^{®} FS 30D, L 30 D-55, L 100-55, L 100, L 12,5, L 12,5 P RL 30 D, RL PO, RL 100, RL 12,5, RS 30 D, RS PO, RS 100, RS 12,5, NE 30 D, NE 40 D, NM 30 D, S 100, S 12,5 S 12,5 P, Kollicoat^{®} MAE 30 DP and Kollicoat^{®} MAE 100 P is used. In various examples, the enteric agent may be a combination of the foregoing solutions or dispersions. In an example, the delayed-release coating includes the enteric agent EUDRAGIT^{®} L 100. In some examples, the tablet or capsule is coated with the enteric agent at a coating weight of about 1-20% such as about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20% coating weight.

### Administration and Dosages

It will be appreciated that the actual dose of the IAP and composition comprising *Bacteroides acidifaciens* to be administered will vary according to the particular compound, the particular dosage form, and the mode of administration. Many factors that may modify the action of the AP-based agent (e.g., body weight, gender, diet, time of administration, route of administration, rate of excretion, condition of the subject, drug combinations, genetic disposition and reaction sensitivities) can be taken into account by those skilled in the art. Administration can be carried out continuously or in one or more discrete doses within the maximum tolerated dose. Optimal administration rates for a given set of conditions can be ascertained by those skilled in the art using conventional dosage administration tests.

Individual doses of the IAP and/or composition comprising *Bacteroides acidifaciens* can be administered in unit dosage forms (e.g., tablets or capsules) containing, for example, from about 0.01 mg to about 1,000 mg, about 0.01 mg to about 900 mg, about 0.01 mg to about 800 mg, about 0.01 mg to about 700 mg, about 0.01 mg to about 600 mg, about 0.01 mg to about 500 mg, about 0.01 mg to about 400 mg, about 0.01 mg to about 300 mg, about 0.01 mg to about 200 mg, from about 0.1 mg to about 100 mg, from about 0.1 mg to about 90 mg, from about 0.1 mg to about 80 mg, from about 0.1 mg to about 70 mg, from about 0.1 mg to about 60 mg, from about 0.1 mg to about 50 mg, from about 0.1 mg to about 40 mg, from about 0.1 mg to about 30 mg, from about 0.1 mg to about 20 mg, from about 0.1 mg to about 10 mg, from about 0.1 mg to about 5 mg, from about 0.1 mg to about 3 mg, or from about 0.1 mg to about 1 mg active ingredient per unit dosage for. For example, a unit dosage form can be about 0.01 mg, about 0.02 mg, about 0.03 mg, about 0.04 mg, about 0.05 mg, about 0.06 mg, about 0.07 mg, about 0.08 mg, about 0.09 mg, about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, about 70 mg, about 71 mg, about 72 mg, about 73 mg, about 74 mg, about 75 mg, about 76 mg, about 77 mg, about 78 mg, about 79 mg, about 80 mg, about 81 mg, about 82 mg, about 83 mg, about 84 mg, about 85 mg, about 86 mg, about 87 mg, about 88 mg, about 89 mg, about 90 mg, about 91 mg, about 92 mg, about 93 mg, about 94 mg, about 95 mg, about 96 mg, about 97 mg, about 98 mg, about 99 mg, about 100 mg, about 200 mg, about 300 mg, about 400 mg, about 500 mg, about 600 mg, about 700 mg, about 800 mg, about 900 mg, or about 1,000 mg of the AP-based agent, inclusive of all values and ranges therebetween.

In one example, the IAP and/or composition comprising *Bacteroides acidifaciens* is administered at an amount of from about 0.01 mg to about 1,000 mg daily, about 0.01 mg to about 900 mg daily, about 0.01 mg to about 800 mg daily, about 0.01 mg to about 700 mg daily, about 0.01 mg to about 600 mg daily, about 0.01 mg to about 500 mg daily, about 0.01 mg to about 400 mg daily, about 0.01 mg to about 300 mg daily, about 0.01 mg to about 200 mg daily, about 0.01 mg to about 100 mg daily, an amount of from about 0.1 mg to about 100 mg daily, from about 0.1 mg to about 95 mg daily, from about 0.1 mg to about 90 mg daily, from about 0.1 mg to about 85 mg daily, from about 0.1 mg to about 80 mg daily, from about 0.1 mg to about 75 mg daily, from about 0.1 mg to about 70 mg daily, from about 0.1 mg to about 65 mg daily, from about 0.1 mg to about 60 mg daily, from about 0.1 mg to about 55 mg daily, from about 0.1 mg to about 50 mg daily, from about 0.1 mg to about 45 mg daily, from about 0.1 mg to about 40 mg daily, from about 0.1 mg to about 35 mg daily, from about 0.1 mg to about 30 mg daily, from about 0.1 mg to about 25 mg daily, from about 0.1 mg to about 20 mg daily, from about 0.1 mg to about 15 mg daily, from about 0.1 mg to about 10 mg daily, from about 0.1 mg to about 5 mg daily, from about 0.1 mg to about 3 mg daily, from about 0.1 mg to about 1 mg daily, or from about 5 mg to about 80 mg daily. In various examples, the IAP is administered at a daily dose of about 0.01 mg, about 0.02 mg, about 0.03 mg, about 0.04 mg, about 0.05 mg, about 0.06 mg, about 0.07 mg, about 0.08 mg, about 0.09 mg, about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, about 70 mg, about 71 mg, about 72 mg, about 73 mg, about 74 mg, about 75 mg, about 76 mg, about 77 mg, about 78 mg, about 79 mg, about 80 mg, about 81 mg, about 82 mg, about 83 mg, about 84 mg, about 85 mg, about 86 mg, about 87 mg, about 88 mg, about 89 mg, about 90 mg, about 91 mg, about 92 mg, about 93 mg, about 94 mg, about 95 mg, about 96 mg, about 97 mg, about 98 mg, about 99 mg, about 100 mg, about 200 mg, about 300 mg, about 400 mg, about 500 mg, about 600 mg, about 700 mg, about 800 mg, about 900 mg, or about 1,000 mg, inclusive of all values and ranges therebetween.

In some examples, a suitable dosage of the IAP and/or composition comprising *Bacteroides acidifaciens* is in a range of about 0.01 mg/kg to about 100 mg/kg of body weight of the subject, about 0.01 mg/kg to about 90 mg/kg of body weight of the subject, about 0.01 mg/kg to about 80 mg/kg of body weight of the subject, about 0.01 mg/kg to about 70 mg/kg of body weight of the subject, about 0.01 mg/kg to about 60 mg/kg of body weight of the subject, about 0.01 mg/kg to about 50 mg/kg of body weight of the subject, about 0.01 mg/kg to about 40 mg/kg of body weight of the subject, about 0.01 mg/kg to about 30 mg/kg of body weight of the subject, about 0.01 mg/kg to about 20 mg/kg of body weight of the subject, about 0.01 mg/kg to about 10 mg/kg of body weight of the subject, for example, about 0.01 mg/kg, about 0.02 mg/kg, about 0.03 mg/kg, about 0.04 mg/kg, about 0.05 mg/kg, about 0.06 mg/kg, about 0.07 mg/kg, about 0.08 mg/kg, about 0.09 mg/kg, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, 1.9 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg body weight, about 20 mg/kg body weight, about 30 mg/kg body weight, about 40 mg/kg body weight, about 50 mg/kg body weight, about 60 mg/kg body weight, about 70 mg/kg body weight, about 80 mg/kg body weight, about 90 mg/kg body weight, or about 100 mg/kg body weight, inclusive of all values and ranges therebetween. In other examples, a suitable dosage of the AP-based agent is in a range of about 0.01 mg/kg to about 10 mg/kg of body weight, in a range of about 0.01 mg/kg to about 9 mg/kg of body weight, in a range of about 0.01 mg/kg to about 8 mg/kg of body weight, in a range of about 0.01 mg/kg to about 7 mg/kg of body weight, in a range of 0.01 mg/kg to about 6 mg/kg of body weight, in a range of about 0.05 mg/kg to about 5 mg/kg of body weight, in a range of about 0.05 mg/kg to about 4 mg/kg of body weight, in a range of about 0.05 mg/kg to about 3 mg/kg of body weight, in a range of about 0.05 mg/kg to about 2 mg/kg of body weight, in a range of about 0.05 mg/kg to about 1.5 mg/kg of body weight, or in a range of about 0.05 mg/kg to about 1 mg/kg of body weight.

In accordance with certain examples, the IAP and/or composition comprising *Bacteroides acidifaciensmay* be administered, for example, more than once daily (e.g., about two, about three, about four, about five, about six, about seven, about eight, about nine, or about ten times per day), about once per day, about every other day, about every third day, about once a week, about once every two weeks, about once every month, about once every two months, about once every three months, about once every six months, or about once every year.

### Methods of Treatment

In various examples, IAP and a composition comprising commensal gut bacteria, including *Bacteroides* acidifaciens, are co-administered. The co-administration can occur simultaneously or sequentially.

In some examples, described are methods of treating or preventing metabolic syndrome, diabetes, hypertension, cardiovascular disease, nonalcoholic fatty liver and other metabolic diseases. In various examples, the metabolic syndrome is associated with elevated triglycerides, elevated low density lipoproteins, reduced high density lipoproteins, reduced lipoprotein index, elevated fasting glucose levels, elevated fasting insulin, reduced glucose clearance following feeding, insulin resistance, impaired glucose tolerance, obesity and combinations thereof. For example, the methods may be used to treat subjects having metabolic syndrome and having abdominal obesity (e.g., waist circumference of 40 inches or above in men or 35 inches or above in women), a blood triglyceride level of 150 mg/dL or greater, HDL of less than 40 mg/dL in men or less than 50 mg/dL in women, systolic blood pressure of 130 mm Hg or greater or diastolic blood pressure of 85 mm Hg or greater and/or fasting glucose of 100 mg/dL or greater. Additional metabolic diseases that may be treated using methods described herein include those described in US2013/0251701, US2011/0206654, and US2004/0115185.

In an example, the metabolic disease is obesity. Early exposure to antibiotics (e.g. within about the first 2 years of life) can disrupt the microbiome and lead to eventual disease. Bailey, et al. JAMA Pediatr. 168(11), Nov 2014 describes how early exposure to antibiotics is linked to obesity. Accordingly, in some examples, the methods protect the microbiome of a child and prevent diseases such as obesity. Accordingly, in some examples, provided are methods for treating or preventing obesity by administering an IAP and/or a composition comprising commensal gut bacteria, including but not limited to *Bacteroides acidifaciens,* of the present invention. Methods described herein retain a normal diversity of bacteria in the intestinal tract, such as for example, Bacteroidetes, Proteobacteria, and Firmicutes, thereby treating or preventing obesity. Further still, IAP and/or commensal gut bacteria, including but not limited to *Bacteroides acidifaciens,* may influence fat absorption at the gastrointestinal tract. Accordingly, provided are methods for treating or preventing obesity by limiting GI fat absorption. In various examples, methods described herein are effective for inducing weight loss or preventing weight gain. In some examples, the subjects may have undertaken or will undertake a surgery of the digestive system; be greater than about 80-100 pounds overweight; have a BMI of greater than about 35 kg/m²; or have a health problem related to obesity. In some examples, the subjects may have dyslipidemia including hyperlipidemia and hyperlipoproteinemia.

In another example, the metabolic disease is diabetes. In various examples, described is a treatment for diabetes (type 1 or type 2) and/or glucose intolerance. In various examples, described is the prevention of diabetes (type 1 or type 2) and/or glucose intolerance. In various examples, described is the reduction of complications from diabetes (type 1 or type 2) and/or glucose intolerance. In some examples, described is a method for treating subjects at risk of diabetes, one or more of insulin resistance, prediabetes, impaired fasting glucose (IFG), and impaired glucose tolerance (IGT).

In various examples, described is the treatment of type 1 diabetes with an IAP. Type 1 diabetes, once known as juvenile diabetes or insulin-dependent diabetes, is a chronic condition in which the pancreas produces little or no insulin. Treatment is often via intensive insulin regimens, which attempt to mimic the body's normal pattern of insulin secretion, and often involve basal and bolus insulin coverage. For example, one common regimen is the administration of a long-acting insulin (including, for example, glargine/detemir) once or twice a day with rapid acting insulin (including, for example, aspart, glulisine, lispro) preprandially or postprandially and as needed to correct high blood sugars (as monitored by a glucose meter, for example). Doses administered preprandially or postprandially or as needed to correct high blood sugars may be referred to as bolus administrations. Another common regimen involves dosing, including continuous dosing, via an insulin pump (or continuous subcutaneous insulin infusion device (CSII)) of, for example a rapid acting insulin (as described herein and including, for example, aspart, glulisine, lispro). In various examples, an IAP may replace any of the insulins used in various regimens, including instances in which the insulins are not providing effective therapy in the patient, an IAP may cause an increase in patient compliance as it may allow for easier self-dosing relative to various forms of insulin, which must be administered as various doses throughout the day- even in the context of an insulin pump, which requires programming. Further, an IAP can offset common frustration of diabetic patient dosing, such as, for example, the dawn phenomenon. Alternatively, an IAP may be used adjuvant to any of the type 1 diabetes treatments described herein to, for example, normalize a patient's regimen and avoid blood sugar "dips" (e.g. hypoglycemia, e.g. blood sugar of below about 70 mg/dL) and "spikes" (e.g. hyperglycemia, e.g. blood sugar of greater than about 200 mg/dL) that afflict many patients. Accordingly, in some examples, an IAP may treat or prevent symptoms associated with hypoglycemia, including for example, shakiness, anxiety, nervousness, palpitations, tachycardia, pallor, coldness, clamminess, dilated pupils (mydriasis), hunger, borborygmus, nausea, vomiting, abdominal discomfort, headache, abnormal mentation, impaired judgment, nonspecific dysphoria, paresthesia, negativism, irritability, belligerence, combativeness, rage, personality change, emotional lability, fatigue, weakness, apathy, lethargy, daydreaming, sleep, confusion, amnesia, lightheadedness or dizziness, delirium, staring, "glassy" look, blurred vision, double vision, flashes of light in the field of vision, automatism, difficulty speaking, slurred speech, ataxia, incoordination, focal or general motor deficit, paralysis, hemiparesis, paresthesia, headache, stupor, coma, abnormal breathing, generalized or focal seizures, memory loss, CNS damage (e.g. cognitive impairment), amnesia, and death. Accordingly, in some examples, an IAP may treat or prevent symptoms associated with hyperglycemia, including for example, polyphagia, polydipsia, polyuria, blurred vision, fatigue, weight loss, poor wound healing, dry mouth, dry or itchy skin, tingling in feet or heels, erectile dysfunction, recurrent infections, external ear infections (e.g. swimmer's ear), cardiac arrhythmia, stupor, coma, and seizures. In various regimens, a type 1 diabetes patient may receive additional agents to supplement insulin therapy. In some examples, an IAP is used in this manner. IAPs may provide additional therapeutic benefits in patients that are struggling to manage type 1 diabetes with insulin therapy alone. In some examples, patients that are struggling to manage type 1 diabetes with insulin therapy alone have poor glycemic control as described herein.

In some examples, an IAP finds use in reducing a patient's blood glucose level to below about 10 mM, e.g. within the range of about 4 mM to about 7 mM.

Described herein is a method for treating type 1 or type 2 diabetes, comprising administering an effective amount of an IAP and composition comprising commensal gut bacteria, including *Bacteroides acidifaciens* to a patient in need thereof.

In a number of examples, including those in which an IAP and composition comprising commensal gut bacteria, including *Bacteroides acidifaciens,* prevents diabetes and/or treats a pre-diabetic condition, a patient is at risk of diabetes if the patient is characterized by one or more of: being physically inactive; having a parent or sibling with diabetes; having a family background associated with high incidence of diabetes, selected from that is African American, Alaska Native, American Indian, Asian American, Hispanic/Latino, or Pacific Islander American; giving birth to a baby weighing more than 9 pounds; being diagnosed with gestational diabetes; having high blood pressure of about 140/90 mmHg or above; being treated for high blood pressure; having HDL cholesterol level below about 35 mg/dL and/ or a triglyceride level above about 250 mg/dL; having polycystic ovary syndrome (PCOS); and having cardiovascular disease.

In various examples, an IAP and composition comprising commensal gut bacteria, including *Bacteroides acidifaciens,* may be used to treat diabetes in the context of hospitalization. For example, an IAP and/or composition comprising commensal gut bacteria, including *Bacteroides acidifaciens,* may be administered to a patient that is in a diabetic coma. In some examples, the patient may be administered to a patient that has one or more of a severe diabetic hypoglycemia, advanced diabetic ketoacidosis (e.g. advanced enough to result in unconsciousness, contributing factors may include one or more of hyperglycemia, dehydration, shock, and exhaustion), hyperosmolar nonketotic coma (e.g. with one or more of hyperglycemia and dehydration are contributing factors). In these examples, an IAP and composition comprising commensal gut bacteria, including *Bacteroides acidifaciens,* may be used in conjunction with standard treatment regimens of diabetic comas, including administering one or more of glucose, glucagon, insulin, fluids (e.g. saline with potassium and/or other electrolytes), any of which, optionally, are administered intravenously. In some examples, an IAP and composition comprising commensal gut bacteria, including *Bacteroides acidifaciens,* may replace insulin in these treatment regimens and, optionally, is administered orally.

In various examples, the IAP and composition comprising commensal gut bacteria, including *Bacteroides acidifaciens,* may be used to treat pregnant women with increased risk of gestational diabetes. Some pregnant women develop gestational diabetes starting around 24-weeks of pregnancy, and if left untreated, gestational diabetes may cause premature birth and still birth. In some examples, provided are methods of preventing and/or treating gestational diabetes in pregnant women. In various examples, methods described herein may also be utilized to treat pregnant women who are at increased risk for inflammation such as GI inflammation. In some examples, the methods reduce inflammation in pregnant women.

Further, in various examples pertaining to diabetes, the patient may be receiving or there may be co-administration with one or more additional agents. Illustrative additional agents include insulin or any anti-diabetic agents (e.g. biguanides, insulin secretogogues such as sulphonylureas or meglitinides, inhibitors of α-glucosidase, thiazolidinediones, and others). The methods of treatment described herein, may comprise administering an IAP and composition comprising commensal gut bacteria, including *Bacteroides acidifaciens,* to a patient that is receiving one or more additional agents and/or non-insulin diabetes agents. Additional agents include one or more of a sulfonylurea (e.g. DYMELOR (acetohexamide), DIABINESE (chlorpropamide), ORINASE (tolbutamide), and TOLINASE (tolazamide), GLUCOTROL (glipizide), GLUCOTROL XL (extended release), DIABETA (glyburide), MICRONASE (glyburide), GLYNASE PRESTAB (glyburide), and AMARYL (glimepiride)); a Biguanide (e.g. metformin (GLUCOPHAGE, GLUCOPHAGE XR, RIOMET, FORTAMET, and GLUMETZA)); a thiazolidinedione (e.g. ACTOS (pioglitazone) and AVANDIA (rosiglitazone); an alpha-glucosidase inhibitor (e.g., PRECOSE (acarbose) and GLYSET (miglitol); a Meglitinide (e.g., PRANDIN (repaglinide) and STARLIX (nateglinide)); a Dipeptidyl peptidase IV (DPP-IV) inhibitor (e.g., JANUVIA (sitagliptin), NESINA (alogliptin), ONGLYZA (saxagliptin), and TRADJENTA (linagliptin)); Sodium-glucose co-transporter 2 (SGLT2) inhibitor (e.g. INVOKANA (canaglifozin)); and a combination pill (e.g. GLUCOVANCE, which combines glyburide (a sulfonylurea) and metformin, METAGLIP, which combines glipizide (a sulfonylurea) and metformin, and AVANDAMET, which uses both metformin and rosiglitazone (AVANDIA) in one pill, KAZANO (alogliptin and metformin), and OSENI (alogliptin plus pioglitazone).

Other additional agents include METFORMIN oral, ACTOS oral, BYETTA subcutaneous, JANUVIA oral, WELCHOL oral, JANUMET oral, glipizide oral, glimepiride oral, GLUCOPHAGE oral, LANTUS subcutaneous, glyburide oral, ONGLYZA oral, AMARYI oral, LANTUS SOLOSTAR subcutaneous, BYDUREON subcutaneous, LEVEMIR FLEXPEN subcutaneous, ACTOPLUS MET oral, GLUMETZA oral, TRADJENTA oral, bromocriptine oral, KOMBIGLYZE XR oral, INVOKANA oral, PRANDIN oral, LEVEMIR subcutaneous, PARLODEL oral, pioglitazone oral, NOVOLOG subcutaneous, NOVOLOG FLEXPEN subcutaneous, VICTOZA 2-PAK subcutaneous, HUMALOG subcutaneous, STARLIX oral, FORTAMET oral, GLUCOVANCE oral, GLUCOPHAGE XR oral, NOVOLOG Mix 70-30 FLEXPEN subcutaneous, GLYBURIDE-METFORMIN oral, acarbose oral, SYMLINPEN 60 subcutaneous, GLUCOTROI XL oral, NOVOLIN R inj, GLUCOTROL oral, DUETACT oral, sitagliptin oral, SYMLINPEN 120 subcutaneous, HUMALOG KWIKPEN subcutaneous, JANUMET XR oral, GLIPIZIDE-METFORMIN oral, CYCLOSET oral, HUMALOG MIX 75-25 subcutaneous, nateglinide oral, HUMALOG Mix 75-25 KWIKPEN subcutaneous, HUMULIN 70/30 subcutaneous, PRECOSE oral, APIDRA subcutaneous, Humulin R inj, Jentadueto oral, Victoza 3-Pak subcutaneous, Novolin 70/30 subcutaneous, NOVOLIN N subcutaneous, insulin detemir subcutaneous, glyburide micronized oral, GLYNASE oral, HUMULIN N subcutaneous, insulin glargine subcutaneous, RIOMET oral, pioglitazone-metformin oral, APIDRA SOLOSTAR subcutaneous, insulin lispro subcutaneous, GLYSET oral, HUMULIN 70/30 Pen subcutaneous, colesevelam oral, sitagliptin-metformin oral, DIABETA oral, insulin regular human inj, HUMULIN N Pen subcutaneous, exenatide subcutaneous, HUMALOG Mix 50-50 KWIKPEN subcutaneous, liraglutide subcutaneous, KAZANO oral, repaglinide oral, chlorpropamide oral, insulin aspart subcutaneous, NOVOLOG Mix 70-30 subcutaneous, HUMALOG Mix 50-50 subcutaneous, saxagliptin oral, ACTOPLUS Met XR oral, miglitol oral, NPH insulin human recomb subcutaneous, insulin NPH and regular human subcutaneous, tolazamide oral, mifepristone oral, insulin aspart protam-insulin aspart subcutaneous, repaglinide-metformin oral, saxagliptin-metformin oral, linagliptin-metformin oral, NESINA oral, OSENI oral, tolbutamide oral, insulin lispro protamine and lispro subcutaneous, pramlintide subcutaneous, insulin glulisine subcutaneous, pioglitazone-glimepiride oral, PRANDIMET oral, NOVOLOG PenFill subcutaneous, linagliptin oral, exenatide microspheres subcutaneous, KORLYM oral, alogliptin oral, alogliptin-pioglitazone oral, alogliptin-metformin oral, and canagliflozin oral.

Other additional agents include Lispro (HUMALOG); Aspart (NOVOLOG); Glulisine (APIDRA); Regular (NOVOLIN R or HUMULIN R); NPH (NOVOLIN N or HUMULIN N); Glargine (LANTUS); Detemir (LEVEMIR); HUMULIN or NOVOLIN 70/30; and NOVOLOG Mix 70/30 HUMALOG Mix 75/25 or 50/50.

Without wishing to be bound by theory, it is believed that IAP and composition comprising commensal gut bacteria, including *Bacteroides acidifaciens,* plays a key role in many gastrointestinal and systemic processes including, for example, participating in intestinal defense, mediating anti-inflammatory functions, maintaining normal gut microflora profiles, maintaining mucosal barrier integrity, and regulating digestion and nutrient (fat) absorption. Accordingly, provided is the use of IAP and composition comprising commensal gut bacteria, including *Bacteroides acidifaciens,* in therapeutic applications for modulating immune functions and metabolic functions. In various examples, provided is the treatment of microbiome-related disorders and metabolic diseases (e.g., metabolic syndrome, obesity, and diabetes).

Described herein are methods for modulating and protecting a patient's gastrointestinal microbiome, comprising administering an effective amount of a pharmaceutical composition comprising an IAP and composition comprising commensal gut bacteria, including *Bacteroides acidifaciens* (and/or additional therapeutic agents), to the patient. In some examples, methods may be used to treat subjects with reduced levels and/or function of gastrointestinal tract flora by administering an IAP and composition comprising commensal gut bacteria, including *Bacteroides acidifaciens* of the invention so as to increase or preserve the number of commensal bacteria and composition of the gastrointestinal microbiome. In other examples, methods relate to treating infections by pathogenic bacteria and/or inhibiting the growth or decrease the number of pathogenic bacteria in the gastrointestinal tract.

In some examples, described herein is the prevention of expansion of the gut resistome. The gut resistome refers to the reservoir of antibiotic resistance genes that may be harbored by the human gut microbiota. Such antibiotic resistance genes confer antibiotic resistance among bacterial pathogens which constitute a major threat to public health. Bacteria can acquire antibiotic resistance genes by the mobilization and transfer of resistance genes from a donor strain. In various examples, the methods reduce the number of antibiotic resistant bacteria in the gastrointestinal tract thereby reducing the expansion of the gut resistome. In various examples, the method described herein mitigates or prevents the growth of antibiotic resistant bacteria thus preventing or diminishing the expansion of the gut resistome.

In various examples, the methods and uses described herein include use of IAP and composition comprising *Bacteroides acidifaciens* as an adjuvant to any of these initial and/or adjunctive therapies (including co-administration or sequential administration). In various examples, the methods and uses described herein include administration of the IAP and composition comprising commensal gut bacteria, including *Bacteroides acidifaciens,* described herein to a subject undergoing initial and/or adjunctive therapies.

In some examples, the terms "patient" and "subject" are used interchangeably. In someexamples, the subject and/or animal is a mammal, e.g., a human, mouse, rat, guinea pig, dog, cat, horse, cow, pig, rabbit, sheep, or non-human primate, such as a monkey, chimpanzee, or baboon. In other examples, the subject and/or animal is a non-mammal, such, for example, a zebrafish.

In various examples, methods described herein are useful in treatment a human patient. In some examples, the human is a pediatric human. In other examples, the human is an adult human. In other examples, the human is a geriatric human. In some examples, the human is a female. In some examples, the human is a male.

In certain examples, the human patient has an age in a range of from about 1 to about 18 months old, from about 18 to about 36 months old, from about 1 to about 5 years old, from about 5 to about 10 years old, from about 10 to about 15 years old, from about 15 to about 20 years old, from about 20 to about 25 years old, from about 25 to about 30 years old, from about 30 to about 35 years old, from about 35 to about 40 years old, from about 40 to about 45 years old, from about 45 to about 50 years old, from about 50 to about 55 years old, from about 55 to about 60 years old, from about 60 to about 65 years old, from about 65 to about 70 years old, from about 70 to about 75 years old, from about 75 to about 80 years old, from about 80 to about 85 years old, from about 85 to about 90 years old, from about 90 to about 95 years old or from about 95 to about 100 years old.

### Additional Therapeutic Agents and Combination Therapy

Administration of the present compositions and formulations comprising the IAP and commensal gut bacteria, including *Bacteroides acidifaciens,* may be combined with additional therapeutic agents. Co-administration of the additional therapeutic agent and the present compositions/formulations may be simultaneous or sequential. Further, the compositions/formulations may comprise an additional therapeutic agent (e.g. via co-formulation). For example, the additional therapeutic agent and the IAP and composition comprising commensal gut bacteria, including *Bacteroides acidifaciens,* may be combined into a single formulation. Alternatively, the additional therapeutic agent and the IAP and composition comprising commensal gut bacteria, including *Bacteroides acidifaciens,* may be formulated separately.

In one example, the additional therapeutic agent and the IAP and composition comprising commensal gut bacteria, including *Bacteroides acidifaciens,* are administered to a subject simultaneously. The term "simultaneously" as used herein, means that the additional therapeutic agent and the IAP and composition comprising commensal gut bacteria, including *Bacteroides acidifaciens,* are administered with a time separation of no more than about 60 minutes, such as no more than about 30 minutes, no more than about 20 minutes, no more than about 10 minutes, no more than about 5 minutes, or no more than about 1 minute. Administration of the additional therapeutic agent and the IAP and composition comprising commensal gut bacteria, including *Bacteroides acidifaciens,* can be by simultaneous administration of a single formulation (e.g., a formulation comprising the additional therapeutic agent and the IAP and composition comprising *Bacteroides acidifaciens)* or of separate formulations (e.g., a first formulation including the additional therapeutic agent and a second formulation including the IAP and composition comprising *Bacteroides acidifaciens).*

In a further example, the additional therapeutic agent and the IAP and composition comprising commensal gut bacteria, including *Bacteroides acidifaciens,* are administered to a subject simultaneously but the release of the additional therapeutic agent and the IAP and composition comprising commensal gut bacteria, including *Bacteroides acidifaciens,* from their respective dosage forms (or single unit dosage form if co-formulated) may occur sequentially.

Co-administration does not require the additional therapeutic agent and the IAP and composition comprising commensal gut bacteria, including *Bacteroides acidifaciens,* to be administered simultaneously, if the timing of their administration is such that the pharmacological activities of the additional therapeutic agent and the IAP and composition comprising commensal gut bacteria, including *Bacteroides acidifaciens,* overlap in time. For example, the additional therapeutic agent and the IAP and composition comprising commensal gut bacteria, including *Bacteroides acidifaciens,* can be administered sequentially. The term "sequentially" as used herein means that the additional therapeutic agent and the IAP and composition comprising commensal gut bacteria, including *Bacteroides acidifaciens,* are administered with a time separation of more than about 60 minutes. For example, the time between the sequential administration of the additional therapeutic agent and the IAP and composition comprising commensal gut bacteria, including *Bacteroides acidifaciens,* can be more than about 60 minutes, more than about 2 hours, more than about 5 hours, more than about 10 hours, more than about 1 day, more than about 2 days, more than about 3 days, or more than about 1 week apart. The optimal administration times will depend on the rates of metabolism, excretion, and/or the pharmacodynamic activity of the additional therapeutic agent and the IAP and composition comprising commensal gut bacteria, including *Bacteroides acidifaciens,* being administered. Either the additional therapeutic agent or the IAP and composition comprising commensal gut bacteria, including *Bacteroides acidifaciens,* may be administered first.

Co-administration also does not require the additional therapeutic agent and the IAP and composition comprising commensal gut bacteria, including *Bacteroides acidifaciens,* to be administered to the subject by the same route of administration. Rather, each therapeutic agent can be administered by any appropriate route, for example, parenterally or non-parenterally.

In some examples, the additional therapeutic agent is an anti-bacterial agent, which includes, but is not limited to, cephalosporin antibiotics (cephalexin, cefuroxime, cefadroxil, cefazolin, cephalothin, cefaclor, cefamandole, cefoxitin, cefprozil, and ceftobiprole); fluoroquinolone antibiotics (cipro, Levaquin, floxin, tequin, avelox, and norflox); tetracycline antibiotics (tetracycline, minocycline, oxytetracycline, and doxycycline); penicillin antibiotics (amoxicillin, ampicillin, penicillin V, dicloxacillin, carbenicillin, vancomycin, and methicillin); monobactam antibiotics (aztreonam); and carbapenem antibiotics (ertapenem, doripenem, imipenem/cilastatin, and meropenem). In some embodiments, the anti-bacterial agent may be any of the penicillin, cephalosporin, monobactam, and carbapenem antibiotics.

In some examples, the additional therapeutic agent is an agent useful for treating obesity. Illustrative agents include, but are not limited to, orlistat, lorcaserin, phentermine-topiramate, naltrexone-bupropion, sibutramine, rimonabant, exenatide, pramlintide, phentermine, benzphetamine, diethylpropion, phendimetrazine, bupropion, and metformin. In various examples, the additional agent is an agent that interferes with the body's ability to absorb specific nutrients in food, such as orlistat, glucomannan, and guar gum. Agents that suppress appetite are also among the additional agents, e.g. catecholamines and their derivatives (such as phentermine and other amphetamine-based drugs), various anti-depressants and mood stabilizers (e.g. bupropion and topiramate), anorectics (e.g. dexedrine, digoxin). Agents that increase the body's metabolism are also among the additional agents. In some examples, additional agents may be selected from among appetite suppressants, neurotransmitter reuptake inhibitors, dopaminergic agonists, serotonergic agonists, modulators of GABAergic signaling, anticonvulsants, antidepressants, monoamine oxidase inhibitors, substance P (NKI) receptor antagonists, melanocortin receptor agonists and antagonists, lipase inhibitors, inhibitors of fat absorption, regulators of energy intake or metabolism, cannabinoid receptor modulators, agents for treating addiction, agents for treating metabolic syndrome, peroxisome proliferator-activated receptor (PPAR) modulators; and dipeptidyl peptidase 4 (DPP-4) antagonists. In some examples, additional agents may be selected from among amphetamines, benzodiazepines, sulfonyl ureas, meglitinides, thiazolidinediones, biguanides, beta-blockers, ACE inhibitors, diuretics, nitrates, calcium channel blockers, phenlermine, sibutramine, lorcaserin, cetilistat, rimonabant, taranabant, topiramate, gabapentin, valproate, vigabatrin, bupropion, tiagabine, sertraline, fluoxetine, trazodone, zonisamide, methylphenidate, varenicline, naltrexone, diethylpropion, phendimetrazine, repaglinide, nateglinide, glimepiride, pioglitazone, rosiglilazone, liraglutide, and sitagliptin.

In an example, the additional therapeutic agent is an agent for treating pre-diabetes, diabetes, type II diabetes, insulin resistance, glucose intolerance, or hyperglycemia. Examples of drugs include, but are not limited to, alpha-glucosidase inhibitors, amylin analogs, dipeptidyl peptidase-4 inhibitors, GLP1 agonists, meglitinides, sulfonylureas, biguanides, thiazolidinediones (TZD), and insulin. Additional examples of such agents include bromocriptine and Welchol. Examples of alpha-glucosidase inhibitors include but are not limited to acarbose and miglitol. An example of an amylin analog is pramlintide. Examples of dipeptidyl peptidase-4 inhibitors include but are not limited to saxagliptin, sitagliptin, vildagliptin, linagliptin, and alogliptin. Examples of GLP1 agonist include but are not limited to liraglutide, exenatide, exenatide extended release. Examples of meglitinides include but are not limited to nateglinide, and repaglinide. Examples of sulfonylureas include but are not limited to chlorpropamide, glimepiride, glipizide, glyburide, tolazamide, and tolbutamide. Examples of biguanides include but are not limited to metformin, Riomet, Glucophage, Glucophage XR, Glumetza. Examples of thiazolidinedione include but are not limited to rosiglitazone and pioglitazone. Examples of insulin include but are not limited to Aspart, Detemir, Glargine, Glulisine, and Lispro. Examples of combination drugs include but are not limited to glipizide/metformin, glyburide/metformin, pioglitazone/glimepiride, pioglitazone/metformin, repaglinide/metformin, rosiglitazone/glimepiride, rosiglitazone/metformin, saxagliptin/metformin, sitagliptin/simvastatin, sitagliptin/metformin, linagliptin/metformin, alogliptin/metformin, and alogliptin/pioglitazone.

### Definitions

As used herein, "a," "an," or "the" can mean one or more than one.

Further, the term "about" when used in connection with a referenced numeric indication means the referenced numeric indication plus or minus up to 10% of that referenced numeric indication. For example, the language "about 50%" covers the range of 45% to 55%.

An "effective amount," when used in connection with medical uses is an amount that is effective for providing a measurable treatment, prevention, or reduction in the rate of pathogenesis of a disorder of interest.

As used herein, something is "decreased" if a read-out of activity and/or effect is reduced by a significant amount, such as by at least about 10%, at least about 20%, at least about 30%, at least about 40%, at least about 50%, at least about 60%, at least about 70%, at least about 80%, at least about 90%, at least about 95%, at least about 97%, at least about 98%, or more, up to and including at least about 100%, in the presence of an agent or stimulus relative to the absence of such modulation. As will be understood by one of ordinary skill in the art, in some embodiments, activity is decreased and some downstream read-outs will decrease but others can increase.

Conversely, activity is "increased" if a read-out of activity and/or effect is increased by a significant amount, for example by at least about 10%, at least about 20%, at least about 30%, at least about 40%, at least about 50%, at least about 60%, at least about 70%, at least about 80%, at least about 90%, at least about 95%, at least about 97%, at least about 98%, or more, up to and including at least about 100% or more, at least about 2-fold, at least about 3-fold, at least about 4-fold, at least about 5-fold, at least about 6-fold, at least about 7-fold, at least about 8-fold, at least about 9-fold, at least about 10-fold, at least about 50-fold, at least about 100-fold, in the presence of an agent or stimulus, relative to the absence of such agent or stimulus.

As referred to herein, all compositional percentages are by weight of the total composition, unless otherwise specified. As used herein, the word "include," and its variants, is intended to be non-limiting, such that recitation of items in a list is not to the exclusion of other like items that may also be useful in the compositions and methods of this technology. Similarly, the terms "can" and "may" and their variants are intended to be non-limiting, such that recitation that an embodiment can or may comprise certain elements or features does not exclude other embodiments of the present technology that do not contain those elements or features.

Although the open-ended term "comprising," as a synonym of terms such as including, containing, or having, is used herein to describe and claim the invention, the present invention, or embodiments thereof, may alternatively be described using alternative terms such as "consisting of' or "consisting essentially of."

As used herein, the words "preferred" and "preferably" refer to embodiments of the technology that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the technology.

The amount of compositions described herein needed for achieving a therapeutic effect may be determined empirically in accordance with conventional procedures for the particular purpose. Generally, for administering therapeutic agents (e.g., microbiome-modulating agents and/or additional therapeutic agents described herein) for therapeutic purposes, the therapeutic agents are given at a pharmacologically effective dose. A "pharmacologically effective amount," "pharmacologically effective dose," "therapeutically effective amount," or "effective amount" refers to an amount sufficient to produce the desired physiological effect or amount capable of achieving the desired result, particularly for treating the disorder or disease. An effective amount as used herein would include an amount sufficient to, for example, delay the development of a symptom of the disorder or disease, alter the course of a symptom of the disorder or disease (e.g., slow the progression of a symptom of the disease), reduce or eliminate one or more symptoms or manifestations of the disorder or disease, and reverse a symptom of a disorder or disease. Therapeutic benefit also includes halting or slowing the progression of the underlying disease or disorder, regardless of whether improvement is realized.

Effective amounts, toxicity, and therapeutic efficacy can be determined by standard pharmaceutical procedures in cell cultures, tissue samples, tissue homogenates or experimental animals, e.g., for determining the LD50 (the dose lethal to about 50% of the population) and the ED50 (the dose therapeutically effective in about 50% of the population). The dosage can vary depending upon the dosage form employed and the route of administration utilized. The dose ratio between toxic and therapeutic effects is the therapeutic index and can be expressed as the ratio LD50/ED50. In some embodiments, compositions and methods that exhibit large therapeutic indices are preferred. A therapeutically effective dose can be estimated initially from in vitro assays, including, for example, cell culture assays or measurements or methane production in stool samples. Also, a dose can be formulated in animal models to achieve a circulating plasma concentration range that includes the IC50 as determined in cell culture, or in an appropriate animal model. Levels of the described compositions in plasma can be measured, for example, by high performance liquid chromatography. The effects of any particular dosage can be monitored by a suitable bioassay. The dosage can be determined by a physician and adjusted, as necessary, to suit observed effects of the treatment.

In certain examples, the effect will result in a quantifiable change of at least about 10%, at least about 20%, at least about 30%, at least about 50%, at least about 70%, or at least about 90%. In some examplesw, the effect will result in a quantifiable change of about 10%, about 20%, about 30%, about 50%, about 70%, or even about 90% or more. Therapeutic benefit also includes halting or slowing the progression of the underlying disease or disorder, regardless of whether improvement is realized.

As used herein, "methods of treatment" are equally applicable to use of a composition for treating the diseases or disorders described herein and/or compositions for use and/or uses in the manufacture of a medicaments for treating the diseases or disorders described herein.

### EXAMPLES

### Example 1: IAP protects and induces expansion of Bacteroides acidifaciens

The ability of IAP to induce favorable expansion of certain gut commensal bacteria (e.g., *Bacteroides acidifaciens)* was assessed in the example.

Two cohorts of wild-type mice received streptomycin (5 mg/ml) for 3 days. Intestinal alkaline phosphatase (IAP) was administered via cage drinking water for 4 days. As a control, a group of mice received vehicle in the cage drinking water for 4 days. Feces were collected before antibiotic administration (at day 0) and at day 4. Feces were subjected to whole genome sequencing and microbiome analysis.

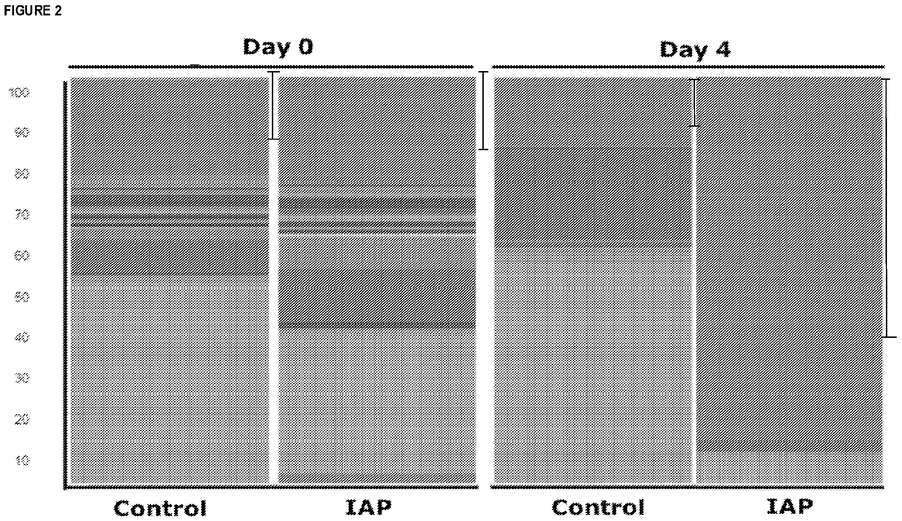

The results depicted in **Figure** 2 show a stacked bar graph of filtered frequency of genus levels. At baseline (day 0) both groups presented similar diversity of gut bacteria. At this time point (day 0), *Bacteroides acidifaciens* was present at frequency of 15% and 18% for the control and IAP groups, respectively. After 3 days of streptomycin treatment, not only was the *Bacteroides acidifaciens* not eliminated by the antibiotic, as seen in the control group where its frequency dropped to 6%, but it became the most predominant bacterium in the microbiome of IAP treated mice (89%).

The data suggests that IAP can favor the survival and expansion of bacteria known to ameliorate metabolic syndrome, weight loss and type 2 diabetes.

### References

J-Y Yang, Y-S Lee, Y Kim, S-H Lee, S Ryu, S Fukuda, K Hase, C-S Yang, HS Lim, M-S Kim, H-M Kim, S-H Ahn, B-E Kwon, H-J Ko and M-N Kweon. (2016). Gut commensal Bacteroides acidifaciens prevents obesity and improves insulin sensitivity in mice. Mucosal Immunology. 10(1): 104-116.

S J Ott, M Musfeldt, D F Wenderoth, J Hampe, O Brant, U R Fo"lsch, K N Timmis, S Schreiber. (2004). Reduction in diversity of the colonic mucosa associated bacterial microflora in patients with active inflammatory bowel disease. Gut. 53:685-693.

## Claims

1. An intestinal alkaline phosphatase (IAP) and a composition comprising at least one commensal gastrointestinal bacteria for use in the treatment or prevention of a metabolic disorder in a patient in need thereof, wherein the IAP and the composition comprising at least one commensal gastrointestinal bacteria are co-administered, wherein the composition comprising at least one commensal gastrointestinal bacteria comprises *Bacteroides acidifaciens,* and wherein the metabolic disorder is obesity, type 1 or type 2 diabetes, and/or a metabolic syndrome.

2. The intestinal alkaline phosphatase (IAP) and the composition comprising at least one commensal gastrointestinal bacteria for use according to claim 1, wherein the composition comprising at least one commensal gut bacteria is a fecal microbiota transplant.

3. The intestinal alkaline phosphatase (IAP) and the composition comprising at least one commensal gastrointestinal bacteria for use according to claim 1, wherein the composition comprising at least one commensal gastrointestinal bacteria is administered simultaneously or sequentially with the IAP.

4. The intestinal alkaline phosphatase (IAP) and the composition comprising at least one commensal gastrointestinal bacteria for use according to claim 2 or 3, wherein the fecal microbiota transplant is stool or a derivative thereof.

5. The intestinal alkaline phosphatase (IAP) and the composition comprising at least one commensal gastrointestinal bacteria for use according to any one of claims 2-4, wherein the fecal microbiota transplant is derived from a human donor.

6. The intestinal alkaline phosphatase (IAP) and the composition comprising at least one commensal gastrointestinal bacteria for use according to any one of the preceding claims, wherein the fecal microbiota transplant is administered via one or more of oral administration, colonoscopy, sigmoidoscopy, enema, naso-gastric intubation, naso-duodenal intubation, and naso-jejunal intubation.

7. The intestinal alkaline phosphatase (IAP) and the composition comprising at least one commensal gastrointestinal bacteria for use according to claim 1, wherein the composition comprising at least one commensal gastrointestinal bacteria is an isolated bacterial composition, optionally wherein the bacteria are isolated from one or more of human stool, the human GI tract, and the human gut, optionally wherein the isolated commensal gastrointestinal bacteria is grown in pure or mixed cultures, optionally wherein the isolated commensal gastrointestinal bacteria is formulated as one or more of tablets, pills, powders, capsules, lyophilized compositions, and aqueous formulations.

8. The intestinal alkaline phosphatase (IAP) and the composition comprising at least one commensal gastrointestinal bacteria for use according to any one of the preceding claims, wherein the IAP is administered orally.

9. The intestinal alkaline phosphatase (IAP) and the composition comprising at least one commensal gastrointestinal bacteria for use according to claim 7, wherein the composition comprising isolated commensal gastrointestinal bacteria is administered orally.

10. The intestinal alkaline phosphatase (IAP) and the composition comprising at least one commensal gastrointestinal bacteria for use according to any one of the preceding claims, wherein the administration of the IAP and the composition comprising at least one commensal gastrointestinal bacteria increases or preserves the number of commensal bacteria and/or composition of the gastrointestinal microbiome of the patient; and/or wherein the administration of the IAP and the composition comprising the at least one commensal gastrointestinal bacteria inhibits the growth of or decreases the number of pathogenic bacteria in the gastrointestinal microbiome of the patient.

11. The intestinal alkaline phosphatase (IAP) and the composition comprising at least one commensal gastrointestinal bacteria for use according to any one of the preceding claims, wherein the treatment of the metabolic disorder comprises amelioration of metabolic syndrome, prevention of diabetes, or prevention of obesity.

12. The intestinal alkaline phosphatase (IAP) and the composition comprising at least one commensal gastrointestinal bacteria for use according to any one of the preceding claims, wherein the IAP comprises an amino sequence having at least about 90%, or about 95%, or about 97%, or about 98%, or about 99% sequence identity to SEQ ID NO: 11.

13. The intestinal alkaline phosphatase (IAP) and the composition comprising at least one commensal gastrointestinal bacteria for use according to claim 12, wherein the IAP is bovine IAP (bIAP).

14. The intestinal alkaline phosphatase (IAP) and the composition comprising at least one commensal gastrointestinal bacteria for use according to claim 13, wherein the bIAP comprises an amino sequence having at least about 97% sequence identity to SEQ ID NO: 11.

## Patentansprüche

1. Intestinale alkalische Phosphatase (IAP) und Zusammensetzung, die mindestens ein kommensales gastrointestinales Bakterium umfasst, zur Verwendung bei der Behandlung oder Prävention einer Stoffwechselstörung bei einem Patienten, der dieser bedarf, wobei die IAP und die Zusammensetzung, die mindestens ein kommensales gastrointestinales Bakterium umfasst, gemeinsam verabreicht werden, wobei die Zusammensetzung, die mindestens ein kommensales gastrointestinales Bakterium umfasst, *Bacteroides acidifaciens* umfasst und wobei die Stoffwechselstörung Adipositas, Typ-1- oder Typ-2-Diabetes und/oder ein metabolisches Syndrom ist.

2. Intestinale alkalische Phosphatase (IAP) und Zusammensetzung, die mindestens ein kommensales gastrointestinales Bakterium umfasst, zur Verwendung nach Anspruch 1, wobei die Zusammensetzung, die mindestens ein kommensales Darmbakterium umfasst, eine fäkale Mikrobiotatransplantation ist.

3. Intestinale alkalische Phosphatase (IAP) und Zusammensetzung, die mindestens ein kommensales gastrointestinales Bakterium umfasst, zur Verwendung nach Anspruch 1, wobei die Zusammensetzung, die mindestens ein kommensales Darmbakterium umfasst, gleichzeitig oder nacheinander mit der IAP verabreicht wird.

4. Intestinale alkalische Phosphatase (IAP) und Zusammensetzung, die mindestens ein kommensales gastrointestinales Bakterium umfasst, zur Verwendung nach Anspruch 2 oder 3, wobei die fäkale Mikrobiotatransplantation Stuhl oder ein Derivat davon ist.

5. Intestinale alkalische Phosphatase (IAP) und Zusammensetzung, die mindestens ein kommensales gastrointestinales Bakterium umfasst, zur Verwendung nach einem der Ansprüche 2-4, wobei die fäkale Mikrobiotatransplantation von einem menschlichen Spender stammt.

6. Intestinale alkalische Phosphatase (IAP) und Zusammensetzung, die mindestens ein kommensales gastrointestinales Bakterium umfasst, zur Verwendung nach einem der vorhergehenden Ansprüche, wobei die fäkale Mikrobiotatransplantation mittels einer bzw. eines oder mehrerer von einer oralen Verabreichung, einer Koloskopie, einer Sigmoidoskopie, einem Einlauf, einer nasogastralen Intubation, einer nasoduodenalen Intubation und einer nasojejunalen Intubation verabreicht wird.

7. Intestinale alkalische Phosphatase (IAP) und Zusammensetzung, die mindestens ein kommensales gastrointestinales Bakterium umfasst, zur Verwendung nach Anspruch 1, wobei die Zusammensetzung, die mindestens ein kommensales gastrointestinales Bakterium umfasst, eine isolierte Bakterienzusammensetzung ist, optional wobei die Bakterien aus einem oder mehreren von einem menschlichen Stuhl, dem menschlichen Magen-Darm-Trakt und dem menschlichen Darm isoliert werden, optional wobei das isolierte kommensale gastrointestinale Bakterium als eine bzw. eines oder mehrere von Tabletten, Pillen, Pulvern, Kapseln, lyophilisierten Zusammensetzungen und wässrigen Formulierungen formuliert wird.

8. Intestinale alkalische Phosphatase (IAP) und Zusammensetzung, die mindestens ein kommensales gastrointestinales Bakterium umfasst, zur Verwendung nach einem der vorhergehenden Ansprüche, wobei die IAP oral verabreicht wird.

9. Intestinale alkalische Phosphatase (IAP) und Zusammensetzung, die mindestens ein kommensales gastrointestinales Bakterium umfasst, zur Verwendung nach Anspruch 7, wobei die Zusammensetzung, die isolierte kommensale gastrointestinale Bakterien umfasst, oral verabreicht wird.

10. Intestinale alkalische Phosphatase (IAP) und Zusammensetzung, die mindestens ein kommensales gastrointestinales Bakterium umfasst, zur Verwendung nach einem der vorhergehenden Ansprüche, wobei die Verabreichung der IAP und der Zusammensetzung, die mindestens ein kommensales gastrointestinales Bakterium umfasst, die Anzahl von kommensalen Bakterien und/oder die Zusammensetzung des gastrointestinalen Mikrobioms des Patienten erhöht oder bewahrt; und/oder wobei die Verabreichung der IAP und der Zusammensetzung, die mindestens ein kommensales gastrointestinales Bakterium umfasst, das Wachstum von pathogenen Bakterien im Magen-Darm-Mikrobiom des Patienten hemmt oder die Anzahl dieser verringert.

11. Intestinale alkalische Phosphatase (IAP) und Zusammensetzung, die mindestens ein kommensales gastrointestinales Bakterium umfasst, zur Verwendung nach einem der vorhergehenden Ansprüche, wobei die Behandlung der Stoffwechselstörung eine Linderung eines metabolischen Syndroms, eine Prävention von Diabetes oder eine Prävention von Adipositas umfasst.

12. Intestinale alkalische Phosphatase (IAP) und Zusammensetzung, die mindestens ein kommensales gastrointestinales Bakterium umfasst, zur Verwendung nach einem der vorhergehenden Ansprüche, wobei die IAP eine Aminosequenz mit einer Sequenzidentität von mindestens etwa 90 % oder etwa 95 % oder etwa 97 % oder etwa 98 % oder etwa 99 % zu SEQ ID Nr. 11 umfasst.

13. Intestinale alkalische Phosphatase (IAP) und Zusammensetzung, die mindestens ein kommensales gastrointestinales Bakterium umfasst, zur Verwendung nach Anspruch 12, wobei die IAP Rinder-IAP (bIAP) ist.

14. Intestinale alkalische Phosphatase (IAP) und Zusammensetzung, die mindestens ein kommensales gastrointestinales Bakterium umfasst, zur Verwendung nach Anspruch 13, wobei die bIAP eine Aminosequenz mit einer Sequenzidentität von mindestens etwa 97 % zu SEQ ID Nr. 11 umfasst.

## Revendications

1. Phosphatase alcaline intestinale (PAI) et composition comprenant au moins une bactérie gastro-intestinale commensale, pour utilisation dans le traitement ou la prévention d'un trouble métabolique chez un patient en ayant besoin, dans lesquelles la PAI et la composition comprenant au moins une bactérie gastro-intestinale commensale sont co-administrées, dans lesquelles la composition comprenant au moins une bactérie gastro-intestinale commensale comprend *Bacteroides acidifaciens,* et dans lesquelles le trouble métabolique est l'obésité, le diabète de type 1 ou type 2 ; et/ou un syndrome métabolique.

2. Phosphatase alcaline intestinale (PAI) et composition comprenant au moins une bactérie gastro-intestinale commensale, pour utilisation selon la revendication 1, dans lesquelles la composition comprenant au moins une bactérie gastro-intestinale commensale est un transplant de microbiote fécal.

3. Phosphatase alcaline intestinale (PAI) et composition comprenant au moins une bactérie gastro-intestinale commensale, pour utilisation selon la revendication 1, dans lesquelles la composition comprenant au moins une bactérie gastro-intestinale commensale est administrée simultanément ou séquentiellement avec la PAI.

4. Phosphatase alcaline intestinale (PAI) et composition comprenant au moins une bactérie gastro-intestinale commensale, pour utilisation selon la revendication 2 ou 3, dans lesquelles le transplant de microbiote fécal consiste en des fèces ou en un dérivé de celles-ci.

5. Phosphatase alcaline intestinale (PAI) et composition comprenant au moins une bactérie gastro-intestinale commensale, pour utilisation selon l'une quelconque des revendications 2-4, dans lesquelles le transplant de microbiote fécal est issu d'un donneur humain.

6. Phosphatase alcaline intestinale (PAI) et composition comprenant au moins une bactérie gastro-intestinale commensale, pour utilisation selon l'une quelconque des revendications précédentes, dans lesquelles le transplant de microbiote fécal est administré via un(e) ou plusieurs parmi administration orale, coloscopie, sigmoïdoscopie, lavement, intubation naso-gastrique, intubation naso-duodénale, et intubation naso-jéjunale.

7. Phosphatase alcaline intestinale (PAI) et composition comprenant au moins une bactérie gastro-intestinale commensale, pour utilisation selon la revendication 1, dans lesquelles la composition comprenant au moins une bactérie gastro-intestinale commensale est une composition bactérienne isolée, en option dans lesquelles les bactéries sont isolées à partir d'une ou de plusieurs sources parmi les fèces humaines, le tractus GI humain, et l'intestin humain, en option dans lesquelles la bactérie gastro-intestinale commensale isolée est cultivée en cultures pures ou mixtes, en option dans lesquelles la bactérie gastro-intestinale commensale isolée est formulée sous forme d'une ou de plusieurs formes parmi les comprimés, pilules, poudres, capsules, compositions lyophilisées, et formulations aqueuses.

8. Phosphatase alcaline intestinale (PAI) et composition comprenant au moins une bactérie gastro-intestinale commensale, pour utilisation selon l'une quelconque des revendications précédentes, dans lesquelles la PAI est administrée par voie orale.

9. Phosphatase alcaline intestinale (PAI) et composition comprenant au moins une bactérie gastro-intestinale commensale, pour utilisation selon la revendication 7, dans lesquelles la composition comprenant des bactéries gastro-intestinales commensales isolées est administrée par voie orale.

10. Phosphatase alcaline intestinale (PAI) et composition comprenant au moins une bactérie gastro-intestinale commensale, pour utilisation selon l'une quelconque des revendications précédentes, dans lesquelles l'administration de la PAI et de la composition comprenant au moins une bactérie gastro-intestinale commensale augmente ou maintient le nombre de bactéries commensales et/ou la composition du microbiome gastro-intestinal du patient; et/ou dans lesquelles l'administration de la PAI et de la composition comprenant ladite au moins une bactérie gastro-intestinale commensale inhibe la croissance de ou diminue le nombre de bactéries pathogènes dans le microbiome gastro-intestinal du patient.

11. Phosphatase alcaline intestinale (PAI) et composition comprenant au moins une bactérie gastro-intestinale commensale, pour utilisation selon l'une quelconque des revendications précédentes, dans lesquelles le traitement du trouble métabolique comprend une atténuation du syndrome métabolique, une prévention du diabète, ou une prévention de l'obésité.

12. Phosphatase alcaline intestinale (PAI) et composition comprenant au moins une bactérie gastro-intestinale commensale, pour utilisation selon l'une quelconque des revendications précédentes, dans lesquelles la PAI comprend une séquence d'acides aminés ayant un degré d'identité de séquence d'au moins environ 90 %, ou environ 95 %, ou environ 97 %, ou environ 98 %, ou environ 99 % avec la séquence SEQ ID NO: 11.

13. Phosphatase alcaline intestinale (PAI) et composition comprenant au moins une bactérie gastro-intestinale commensale, pour utilisation selon la revendication 12, dans lesquelles la PAI est la PAI bovine (PAlb).

14. Phosphatase alcaline intestinale (PAI) et composition comprenant au moins une bactérie gastro-intestinale commensale, pour utilisation selon la revendication 13, dans lesquelles la PAIb comprend une séquence d'acides aminés ayant un degré d'identité de séquence d'au moins environ 97 % avec la séquence SEQ ID NO: 11.
